# EUROPEAN PATENT SPECIFICATION

(11) **EP 4 130 287 B1**
(45) Date of publication and mention of the grant of the patent: **28.01.2026**
(21) Application number: 20929208.5
(22) Date of filing: 25.09.2020
(51) Int. Cl.: G01N 33/533, G01N 33/50, C12N 5/00, G01N 1/30

(54) **METHOD FOR EVALUATING SAMPLE CONTAINING CELL SUPPORT-DERIVED COMPONENT**
VERFAHREN ZUR BEURTEILUNG EINER PROBE MIT EINER ZELLTRÄGERABGELEITETEN KOMPONENTE
PROCÉDÉ D'ÉVALUATION D'UN ÉCHANTILLON CONTENANT UN COMPOSANT DÉRIVÉ D'UN SUPPORT CELLULAIRE

(30) Priority: 31.03.2020 JP 2020062243
(43) Date of publication of application: 08.02.2023
(73) Proprietor: Minaris Advanced Therapies Co., Ltd., Yokohama, Kanagawa 221-0024 (JP)
(72) Inventor: MARUYAMA Masashi, Tokyo 100-8280 (JP); SAKUMA Hirotaka, Tokyo 100-8280 (JP); SATO Yushi, Tokyo 100-6606 (JP); TADA Yasuhiko, Tokyo 100-8280 (JP)
(74) Representative: Winter, Brandl - Partnerschaft mbB
(86) International application number: PCT/JP2020/036253
(87) International publication number: WO 2021/199459

(56) References cited:
- JP-A- 2019 152 604
- CHEN RUYING ET AL: "Scaffold-mediated delivery for non-viral mRNA vaccines", GENE THERAPY, NATURE PUBLISHING GROUP, LONDON, GB, vol. 25, no. 8, 21 September 2018 (2018-09-21), pages 556 - 567, XP037114870, ISSN: 0969-7128, [retrieved on 20180921], DOI: 10.1038/S41434-018-0040-9
- OMAR S. ALJITAWI ET AL: "A novel three-dimensional stromal-based model for in vitro chemotherapy sensitivity testing of leukemia cells", LEUKEMIA AND LYMPHOMA., vol. 55, no. 2, 26 February 2014 (2014-02-26), US, pages 378 - 391, XP055368038, ISSN: 1042-8194, DOI: 10.3109/10428194.2013.793323
- MILIND SINGH ET AL: "Microsphere-Based Seamless Scaffolds Containing Macroscopic Gradients of Encapsulated Factors for Tissue Engineering", TISSUE ENGINEERING PART C: METHODS, vol. 14, no. 4, 1 December 2008 (2008-12-01), pages 299 - 309, XP055013057, ISSN: 1937-3384, DOI: 10.1089/ten.tec.2008.0167
- SHKAND TATIANA V ET AL: "Assessment of alginate hydrogel degradation in biological tissue using viscosity-sensitive fluorescent dyes", vol. 4, no. 4, 28 September 2016 (2016-09-28), pages 044002, XP093062298, Retrieved from the Internet <URL:http://stacks.iop.org/2050-6120/4/i=4/a=044002?key=crossref.9c6818e023e4823cf3c44a7c388e8db8> DOI: 10.1088/2050-6120/4/4/044002
- OWENS ERIC A ET AL: "Highly charged cyanine fluorophores for trafficking scaffold degradation", vol. 8, no. 1, 25 January 2013 (2013-01-25), GB, pages 014109, XP093062303, ISSN: 1748-6041, Retrieved from the Internet <URL:https://iopscience.iop.org/article/10.1088/1748-6041/8/1/014109> DOI: 10.1088/1748-6041/8/1/014109
- NATALIE ARTZI ET AL: "In vivo and in vitro tracking of erosion in biodegradable materials using non-invasive fluorescence imaging", NATURE MATERIALS, vol. 10, no. 9, 1 January 2011 (2011-01-01), London, pages 704 - 709, XP055510330, ISSN: 1476-1122, DOI: 10.1038/nmat3095
- CUNHA-REIS CASSILDA ET AL: "Fluorescent labeling of chitosan for use in non-invasive monitoring of degradation in tissue engineering : Fluorescent chitosan for monitoring of degradation", JOURNAL OF TISSUE ENGINEERING AND REGENERATIVE MEDICINE, vol. 7, no. 1, 28 November 2011 (2011-11-28), US, pages 39 - 50, XP093062342, ISSN: 1932-6254, DOI: 10.1002/term.494
- KIM SOON HEE ET AL: "Near-Infrared Fluorescence Imaging for Noninvasive Trafficking of Scaffold Degradation", vol. 3, no. 1, 5 February 2013 (2013-02-05), XP093062345, Retrieved from the Internet <URL:https://www.nature.com/articles/srep01198.pdf> DOI: 10.1038/srep01198
- LUH B S ET AL: "End products and mechanism of hydrolysis of pectin and pectic acid by yeast polygalacturonase (YPG)", ARCHIVES OF BIOCHEMISTRY AND BIOPHYSICS, ACADEMIC PRESS, US, vol. 51, no. 1, 1 July 1954 (1954-07-01), pages 102 - 113, XP024810139, ISSN: 0003-9861, [retrieved on 19540701], DOI: 10.1016/0003-9861(54)90458-0
- LIU QINXIANG ET AL: "Pectin Methylesterase and Pectin Remodelling Differ in the Fibre Walls of Two Gossypium Species with Very Different Fibre Properties", PLOS ONE, vol. 8, no. 6, 5 June 2013 (2013-06-05), pages e65131, XP093031118, DOI: 10.1371/journal.pone.0065131
- WARKIANI M. EBRAHIMI, R. MOLOUDI , S. OH & M. WIN NAING: "Intertial-based Microcarrier-cell retention in bioprocessing", CYTOTHERAPY, 1 May 2019 (2019-05-01), pages e4 - e5, XP055936048, [retrieved on 20220628]
- KALRA K., B. BANERJEE , K. WEISS & C. MORGAN: "Developing efficient bioreactor microcarrier cell culture system for large scale production of mesench ymal stem cells (MSCs)", CYTOTHERAPY, 1 May 2019 (2019-05-01), pages s73, XP055936051, [retrieved on 20220628]

## Description

### TECHNICAL FIELD

The disclosure relates to a method for measuring cell support-derived components.

### BACKGROUND ART

In recent years, large-scale culture of cells is being conducted for the purpose of using those cells in fields such as regenerative medicine. In order to achieve efficient large-scale culture of cells, cell supports such as spherical microcarriers are sometimes used as scaffolds during cell proliferation. For example, JP 2018-520662 A discloses microcarriers that is a digestible substrate composed mainly of a polygalacturonic acid, and discloses that during harvesting of the cells, the microcarriers are digested, enabling the cells to be separated from the microcarriers. Tristan Lawson et al. disclose a method for large-scale culture of cells using microcarriers, wherein the cells are separated from the microcarriers using a filter that allows passage of the cells but retains the microcarriers (Tristan Lawson et al. "Process development for expansion of human mesenchymal stromal cells in a 50 L single-use stirred tank bioreactor", Biochemical Engineering Journal, 120 (2017), pp. 49 to 62).

Chen Ruying et al. describe a way to use implantable porous scaffolds as a local gene delivery depot to enhance mRNA vaccine immunization *in vitro,* and compared them *in vivo* with conventional bolus injections (Chen Ruying et al.: "Scaffold-mediated delivery for non-viral mRNA vaccines", Gene Therapy, vol. 25, 8, 21 (2018)).Omar S. Aljitawi et al. disclose a three-dimensional stromal-based model for *in vitro* chemotherapy sensitivity testing of leukemia cells (Omar S. Aljitawi et al.: "A novel three-dimensional stromal-based model for in vitro chemotherapy sensitivity testing of leukemia cells", Leukemia and Lymphoma., vol. 55, no. 2 (2014)).
Milind Singh et al. describe a microparticle-based scaffold fabrication technique as a method to create 3D scaffolds with spatial control over model dyes using uniform poly(D,L-lactide-co-glycolide) microspheres. (Milind Singh et al: "Microsphere-Based Seamless Scaffolds Containing Macroscopic Gradients of Encapsulated Factors for Tissue Engineering", Tissue Engineering Part C: Methods, vol. 14, no. 4 (2008)).
Shkand Tatiana V et al. investigate viscosity-sensitive and viscosity-insensitive fluorescent dyes to distinguish different rheological states of hydrogel based biostructural materials and carriers in biological tissues and to assess their corresponding location areas. (Shkand Tatiana V et al.: "Assessment of alginate hydrogel degradation in biological tissue using viscosity-sensitive fluorescent dyes", Methods and applications in fluorescence, vol. 4, no. 4 (2016)).
Owens Eric A et al. show that the noninvasive optical imaging using near-infrared fluorophores can be useful for the translation of biodegradable scaffolds into the clinic (Owens Eric A et al.: "Highly charged cyanine fluorophores for trafficking scaffold degradation", Biomedical Materials, vol. 8, no. 1 (2013)).

Natalie Artzi et al. harnessed non-invasive fluorescence imaging to sequentially follow *in vivo* material-mass loss in order to model the degradation of materials hydrolytically (PEG:dextran hydrogel) and enzymatically (collagen). (Natalie Artzi et al.: "In vivo and in vitro tracking of erosion in biodegradable materials using non-invasive fluorescence imaging", Nature Materials, vol. 10, no. 9 (2011)). This approach resulted in rapid *in vitro* screening of materials, and can be extended to simultaneously determine drug release and material erosion from a drug-eluting scaffold, or cell viability and material fate in tissue-engineering formulations.
Cunha-Reis Cassilda et al. disclose a method of fluorescent labeling of chitosan for use in non-invasive monitoring of degradation in tissue engineering (Cunha-Reis Cassilda et al.: "Fluorescent labeling of chitosan for use in non-invasive monitoring of degradation in tissue engineering : Fluorescent chitosan for monitoring of degradation", Journal of tissue engineering and regenerative medicine, vol. 7, no. 1 (2011)).
Kim Soon Hee et al. disclose functional biodegradable scaffolds by employing invisible near-infrared fluorescence and followed their degradation behaviors *in vitro* and *in vivo* (Kim Soon Hee et al.: "Near-lnfrared Fluorescence Imaging for Noninvasive Trafficking of Scaffold Degradation", Scientific Reports, vol. 3, no. 1 (2013)). Thereby, they optimized the *in vitro* process of enzyme-based biodegradation to predict implanted scaffold behaviors *in vivo,* which was closely related to the site of inoculation.
Luh B. S.et al. describe rapid hydrolysis rate of pectic acid by yeast polygalacturonase (YPG), wherein about 25 % of the glycosidic bonds are hydrolyzed (Luh B. Set al.: "End products and mechanism of hydrolysis of pectin and pectic acid by yeast polygalacturonase (YPG)", Archives of Biochemistry and Biophysics, vol. 51, no. 1 (1954)).
Liu Qinxiang et al. investigate differences in pectin remodeling during the transition of wall thickening and its influence on final fibre diameter and length during cell expansion in plants (Liu Qinxiang et al.: "Pectin Methylesterase and Pectin Remodelling Differ in the Fibre Walls of Two Gossypium Species with Very Different Fibre Properties", PLOS ONE, vol. 8, no. 6, (2013).

### SUMMARY OF INVENTION

### PROBLEMS INVENTION AIMS TO SOLVE

When using cultivated cells, the cell support is digested or dissolved to enable collection of the cells that have separated from the cell support. However, residues from the cell support may sometimes be collected together with the cells. Even in methods where a filter is used to separate the cells from the microcarriers, residues of the microcarriers are sometimes collected together with the cells.

The disclosure has an object of providing a method for measuring cell support-derived components in a liquid sample, the cell support-derived components are derived from a polysaccharide cell support used in cell culture, that enables simple measurement of the cell support-derived components.

### MEANS FOR SOLUTION OF THE PROBLEMS

This object is solved by the subject-matter of the independent claim. Further aspects are disclosed in the subclaims. The invention is thus defined by the claims.

### EFFECTS OF THE INVENTION

The disclosure is able to provide a method for measuring cell support-derived components in a liquid sample, wherein the cell support-derived components are derived from a polysaccharide cell support used in cell culture, that enables simple measurement of the cell support-derived components.

### BRIEF DESCRIPTION OF THE DRAWINGS

FIG. 1 illustrates bright field images and fluorescent images of cell suspensions in Production Example 2.
FIG. 2 illustrates bright field images and fluorescent images of cell suspensions in Production Example 2.
FIG. 3 illustrates bright field images and fluorescent images of cell suspensions in Production Example 2.
FIG. 4 illustrates bright field images and fluorescent images of cell suspensions in Production Example 3.
FIG. 5 is a flowchart illustrating one example of a method for evaluating microcarriers.
FIG. 6 is a flowchart illustrating one example of a method for evaluating a cell suspension.

### EMBODIMENTS FOR CARRYING OUT THE INVENTION

Embodiments of the disclosure are described below, but the disclosure is not limited to these embodiments. Embodiments are embodiments of the invention only if they fall within the scope of the claims. Otherwise they are mere embodiments of the disclosure.

A method according to one of the embodiments is characterized by measuring cell support-derived components in a liquid sample, wherein the cell support-derived components are derived from a polysaccharide cell support used in cell culture, based on optical information.

A method according to one embodiment is a method for measuring cell support-derived components in a liquid sample such as a cell suspension containing cells, an evaluation sample obtained from a cell suspension, a sample containing a liquid and microcarriers for use in cell culture, and a sample containing a liquid obtained following treatment of microcarriers, wherein the cell support-derived components are derived from a polysaccharide cell support used in cell culture, and the method comprises preparing a mixture containing the liquid sample, an acid and at least one aromatic compound that can produce a fluorescent or light-absorbing compound as a result of a reaction with a saccharide under heating, wherein the aromatic compound has at least one functional group selected from the group consisting of a hydroxyl group, an amino group, a nitro group and a carbonyl group, heating the mixture, and subjecting a reaction product in the mixture following heating to fluorescence analysis or absorption analysis.

In this description, a cell support may also be referred to as microcarriers or a culture carrier, and is a moiety that functions as a scaffold for cell growth. The cell support may be either a dissolvable cell support or an indissolvable cell support. Dissolvable cell supports can be decomposed and removed using an enzyme or the like. Indissolvable cell supports can be physically removed using a filter or the like. Polysaccharide cell supports can be decomposed and removed using an enzyme or the like, and can be treated as dissolvable cell supports. Polymers having a hydrophobic portion are difficult to decompose and remove, and can therefore be treated as indissolvable cell support materials.

One example of this method is a measurement method that includes measuring cell support-derived components in a liquid sample, wherein the cell support-derived components are derived from a polysaccharide cell support used in cell culture, using a mixture containing polysaccharide cell support-derived components, an acid, and at least one an aromatic compoundthat can produce a fluorescent or light-absorbing compound as a result of a reaction with a saccharide under heating and wherein the aromatic compound has at least one functional group selected from the group consisting of a hydroxyl group, an amino group, a nitro group and a carbonyl group. More detailed descriptions are presented below as measurement methods 1 and 2.

Another example of this method is a method for measuring a cell suspension containing cells that are cultured in the presence of a dissolvable cell support and collected following dissolution of the cell support, wherein the method includes measuring the cell suspension using a mixture containing the liquid sample, an acid, and at least one aromatic compound that can produce a fluorescent or light-absorbing compound as a result of a reaction with a saccharide under heating, wherein the aromatic compound has at least one functional group selected from the group consisting of a hydroxyl group, an amino group, a nitro group and a carbonyl group. More detailed descriptions are presented below as evaluation methods 1 and 2.

Yet another example of this method is a method that includes measuring a cell suspension, the method including adding a fluorescent dye that is capable of staining a polymer having a hydrophobic portion to a cell suspension containing cells, irradiating the cell suspension containing the added fluorescent dye with an excitation light and performing fluorescence analysis, and then evaluating the cell suspension based on the fluorescence analysis. A more detailed description is presented below as a cell suspension evaluation method.

Yet another example of this method is a method that includes measuring microcarriers, the method including preparing a sample which either contains a liquid and microcarriers for use in cell culture, or contains a liquid obtained following treatment of microcarriers, adding a fluorescent dye that is capable of staining a polymer having a hydrophobic portion to the sample and performing fluorescence analysis, and evaluating the microcarriers based on the fluorescence analysis. A more detailed description is presented below as microcarriers evaluation method.

### (First Embodiment)

### <Measurement Methods>

A method for measuring polysaccharide cell support-derived components in a liquid sample, wherein the cell support-derived components are derived from a polysaccharide cell support used in cell culture, according to one embodiment includes heating a mixture containing the liquid sample, an acid, and at least one aromaticthat can produce a fluorescent or light-absorbing compound as a result of a reaction with a saccharide under heating, the aromatic compound can produce a fluorescent or light-absorbing compound as a result of a reaction with a saccharide under heating is resorcinol, orcinol, fluoroglucinol, naphthoresorcinol, 5-oxytetralone, or anthrone, and wherein the aromatic compound has at least one functional group selected from the group consisting of a hydroxyl group, an amino group, a nitro group and a carbonyl group, heating the mixture, and subjecting the reaction product in the mixture following heating to fluorescence analysis or absorption analysis (hereafter sometimes referred to as the measurement method 1).

A method for measuring polysaccharide cell support-derived components in a liquid sample, wherein the cell support-derived components are derived from a polysaccharide cell support used in cell culture, according to another embodiment includes heating a mixture containing the liquid sample, an acid, and at least one aromatic compound that can produce a fluorescent or light-absorbing compound as a result of a reaction with a saccharide under heating, wherein the aromatic compound has at least one functional group selected from the group consisting of a hydroxyl group, an amino group, a nitro group and a carbonyl group, heating the mixture, subjecting a reaction product in the mixture following heating to fluorescence analysis or absorption analysis and quantifying the reaction product in the mixture following heating, wherein the mass of the aromatic compound is at least 200 times the mass of the polysaccharide cell support-derived components (hereafter sometimes referred to as the measurement method 2).

By using the measurement method 1, the reaction product in the mixture obtained by heating the cell support-derived components in a liquid sample, wherein the cell support-derived components are derived from a polysaccharide cell support used in cell culture, together with an acid and a specific organic compound are analyzed by fluorescence analysis or absorption analysis, and therefore the cell support-derived components in a liquid sample, wherein the cell support-derived components are derived from a polysaccharide cell support used in cell culture, can be measured easily.

By using the measurement method 2, the reaction product in the mixture obtained by heating the cell support-derived components in a liquid sample, wherein the cell support-derived components are derived from a polysaccharide cell support used in cell culture, together with an acid and an excess of a specific organic compound are quantified, and therefore the cell support-derived components in a liquid sample, wherein the cell support-derived components are derived from a polysaccharide cell support used in cell culture, can be measured easily.

The measurement method 1 and the measurement method 2 are sometimes jointly referred to as simply "the measurement method".

In this description, a cell support may also be referred to as microcarriers or a culture carrier, and is a moiety that functions as a scaffold for cell growth. In this embodiment, a dissolvable cell support is used. The term "dissolvable cell support(s)" means that the cell support(s) can be decomposed using an enzyme or the like to a degree that enables release of adhered cells from the cell support. There are no particular limitations on the material of the dissolvable cell support, but specific examples include polygalacturonic acids such as pectin and pectates, and polysaccharides such as alginates, cellulose, crosslinked agarose, dextran and chitosan. In this description, a cell support containing a polysaccharide is sometimes referred to as a polysaccharide cell support. Examples of the form of the cell support include beads, fibers, meshes, nonwoven fabrics and sponges. The amount of the cell support varies depending on the type of cell, but is typically used in an amount within a range from 0.1 g/L to 100 g/L, from 1 g/L to 100 g/L, from 1 g/L to 50 g/L, or from 1.0 g/L to 20 g/L.

The dissolvable cell supports can be dissolved in aqueous media such as culture media and physiological saline solution, depending on the conditions employed, which are selected in accordance with the composition of the cell support, and for example, the cell support can be decomposed by a degrading enzyme. In one embodiment, a conventional hydrolase such as an arginase, pectinase or dextranase that hydrolyzes the polysaccharide that constitutes the cell support may be used.

The measurement method of the disclosure includes heating a mixture containing polysaccharide cell support-derived components in a liquid sample, an acid, and at least one aromatic that can produce a fluorescent or light-absorbing compound as a result of a reaction with a saccharide under heating, wherein the aromatic compound has at least one functional group selected from the group consisting of a hydroxyl group, an amino group, a nitro group and a carbonyl group (hereafter sometimes referred to as simply "the specified aromatic compound").

The term "polysaccharide cell support-derived component(s)" means a component obtained upon dissolution of the dissolvable polysaccharide cell support(s) using a dissolution method selected in accordance with the type of cell support. For example, monosaccharides or oligosaccharides or the like obtained by decomposition of the polysaccharide with a hydrolase correspond with cell support-derived components in a liquid sample, wherein the cell support-derived components are derived from a polysaccharide cell support used in cell culture.

There are no particular limitations on the amount of the polysaccharide cell support-derived components in a liquid sample, wherein the cell support-derived components are derived from a polysaccharide cell support used in cell culture, in the mixture, and the amount may be set appropriately in accordance with factors such as the type of polysaccharide cell support-derived component and the type of the specified aromatic compound. In one example, from the viewpoint of measurement efficiency or sensitivity or the like, the amount of the polysaccharide cell support-derived components in the mixture may be within a range from 0.1 µg/mL to 1,000 µg/mL, from 0.5 µg/mL to 500 µg/mL, or from 0.8 µg/mL to 100 µg/mL. The amount of the polysaccharide cell support-derived components in a liquid sample, wherein the cell support-derived components are derived from a polysaccharide cell support used in cell culture, can be determined, for example, from the amount of the cell support used in the cell culture. From the viewpoint of measurement efficiency and the like, if necessary, the amount may be adjusted so as to satisfy the above concentration range.

For the acid, strong acids such as hydrochloric acid, sulfuric acid and sulfonic acid may be used. The acid has the function of promoting the reaction between the cell support-derived components in a liquid sample, wherein the cell support-derived components are derived from a polysaccharide cell support used in cell culture, and the specified aromatic compound within the mixture that generates the reaction product (colored substance) that represents the measurement target, and in some cases, may also have a function of promoting the decomposition of the polysaccharide cell support in the mixture to the cell support-derived components in a liquid sample, wherein the cell support-derived components are derived from a polysaccharide cell support used in cell culture. Decomposition of the polysaccharide cell support may be promoted by preparing a mixture of the polysaccharide cell support and the acid in advance. Appropriate operations may be conducted in a stepwise manner in accordance with the type of specified aromatic compound being used.

The concentration of the acid in the mixture, expressed as an amount of added acid, may be within a range from 0.01 mol/L to 20 mol/L, from 0.1 mol/L to 12 mol/L, from 0.5 mol/L to 10 mol/L, or from 1.0 mol/L to 10 mol/L.

The specified aromatic compound can produce a fluorescent or light-absorbing compound as a result of a reaction with a saccharide under heating, namely, a monocyclic or polycyclic aromatic compound capable of producing a compound having a functional group that can function as a chromophore. The functional group selected from the group consisting of a hydroxyl group, an amino group, a nitro group and a carbonyl group within the specified aromatic compound may be a single functional group, or a plurality of such functional groups may exist. In the case of a plurality of functional groups, a plurality of the same type of functional group may exist, or an arbitrary combination of different types of functional groups may exist. The aromatic compound is a compound having an aromatic ring, and the aromatic ring may be a hydrocarbon ring, a heteroaromatic ring, or a condensed polycyclic structure that contains an aromatic ring. Representative examples of aromatic hydrocarbon rings include a benzene ring, naphthalene ring, anthracene ring, tetralin ring and hexahydroanthracene ring. Examples of heteroaromatic rings include nitrogen heterocycles such as a carbazole ring and an indole ring.

Among these types of specified aromatic compounds, examples of aromatic compounds having a hydroxyl group include monophenols and polyphenols, and the number of hydroxyl groups is preferably from one to about three. Examples of aromatic compounds having an amino group include primary aromatic amines and secondary aromatic amines, and include not only monoamines, but also polyamines containing two or more primary amino groups and/or secondary amino groups. The secondary amine may be a heterocyclic amine structure in which the nitrogen atom is contained within a heterocyclic ring. Examples of aromatic compounds having a nitro group include aromatic nitro compounds in which one or more nitro groups are substituted to an aromatic ring. Examples of aromatic compounds having a carbonyl group include condensed polycyclic aromatic compounds containing a cyclic ketone group.

More specific examples of the specified aromatic compound include aromatic primary amine compounds such as aniline, p-anisidine, α-naphthylamine and o-dianisidine; aromatic secondary amine compounds such as diphenylamine, carbazole, indole, tryptophan and skatole; phenol compounds such as α-naphthol, resorcinol, orcinol, fluoroglucinol, naphthoresorcinol and 5-oxytetralone; aromatic carbonyl compounds such as anthrone; and nitro compounds such as 3,5-dinitrosalicylic acid, 3,4-dinitrobenzoic acid, m-dinitrobenzene, o-dinitrobenzene and 2,4-dinitrophenol. These compounds may exist within the mixture during reaction, or may be produced prior to the reaction.

In one embodiment, an aromatic compound having at least one hydroxyl group, amino group or cyclic ketone group may be used. From the viewpoint of sensitivity, the compound used as the aromatic compound having at least one hydroxyl group, amino group or cyclic ketone group may be a phenol compound such as resorcinol, orcinol, fluoroglucinol, naphthoresorcinol and 5-oxytetralone, or an aromatic ketone compound such as anthrone, and among these, naphthoresorcinol may be used particularly favorably. Naphthoresorcinol is known to react with saccharides such as monosaccharides (in a reaction that is sometimes called the naphthoresorcinol reaction) to produce colored substances.

The concentration of the specified aromatic compound in the mixture may be selected appropriately in accordance with factors such as the type of specified aromatic compound selected to produce the reaction product, and the type of reaction product that can function as the measurement target.

In the measurement method 1, as mentioned above, there are no particular limitations on the amount of the specified aromatic compound in the mixture, but in one example, from the viewpoint of the measurement sensitivity, the amount may be within a range from 0.1 mg/mL to 100 mg/mL, or from 1 mg/mL to 50 mg/mL.

In the measurement method 2, the amount of the specified aromatic compound in the mixture may be any amount that is at least 200 times the mass of the polysaccharide cell support-derived components in a liquid sample, wherein the cell support-derived components are derived from a polysaccharide cell support used in cell culture, and this amount may be within a range from 200 to 20,000 times, from 250 to 10,000 times, or from 300 to 5,000 times. In this manner, production of the reaction product can be achieved by ensuring that the mixture contains an excess of the specified aromatic compound. The reaction product produced varies depending on the type of the selected aromatic compound and the like, but in those cases where naphthoresorcinol is selected as the specified aromatic compound, the obtained reaction product exhibits fluorescence or light absorption, and has the property of being indissolvable in organic solvents such as butyl acetate or benzene, but dissolvable in alcohols such as ethanol, although this solubility property is not a particular limitation. The amount of the specified aromatic compound in the measurement method 2 may be selected in accordance with factors such as the type of specified aromatic compound and the type of saccharide, but may be at least 20 times, at least 50 times, or at least 100 times the mass of the polysaccharide cell support-derived components in a liquid sample, wherein the cell support-derived components are derived from a polysaccharide cell support used in cell culture.

The mixture containing the polysaccharide cell support-derived components in a liquid sample, wherein the cell support-derived components are derived from a polysaccharide cell support used in cell culture, the acid and at least one aromatic compound that can produce a fluorescent or light-absorbing compound as a result of a reaction with a saccharide under heating may also contain an aqueous solvent. Examples of this aqueous solvent include water, physiological saline solution, and phosphate buffered physiological saline solution (PBS) and the like.

The heating of the mixture may be conducted at 70°C to 120°C, 80°C to 110°C, or 80°C to 100°C, for a period of 10 minutes to 24 hours, 30 minutes to 12 hours, or 1 hour to 8 hours. Stirring may be performed during the heating. As a result of this process, a reaction product corresponding with the type of specified aromatic compound that has been selected is produced in the mixture. The mixture may be cooled following heating, and for example, may be cooled to not more than 60°C, not more than 40°C, or to room temperature (18°C to 28°C).

The measurement method 1 includes subjecting the reaction product in the mixture following heating to fluorescence analysis or absorption analysis.

In the measurement method 1, the fluorescence analysis or absorption analysis of the reaction product may be conducted in the typical type of manner used for such analyses. For example, the analysis may be conducted in the following manner. Namely, the produced reaction product is cooled to room temperature, an organic solvent that is immiscible with water is added to the reaction product, and following agitation, the water phase is removed, yielding a mixture of the organic phase and indissolvable matter. An alcohol is then added to the obtained mixture and stirred to obtain a uniform solution. The obtained solution is then further diluted with alcohol, and the resulting dilute solution can then be used for analysis. Examples of organic solvents that may be used include butyl acetate and benzene and the like. Examples of alcohols that may be used include ethanol and the like.

Either fluorescence analysis or absorption analysis are used as the method of analysis. The method of analysis may be selected in accordance with the coloration wavelength and the like of the obtained reaction product. The analysis may comprise not only detection, but also quantification, of the reaction product.

Specifically, the analysis may be performed, for example, in the manner described below. First, a plurality of simulated dilute solutions having known concentrations of the monosaccharide or saccharide (hereafter jointly referred to as the "saccharide") are prepared, the saccharide concentration and the fluorescent intensity of the simulated dilute solution are measured for each of these simulated dilute solutions, and based on the observed relationship between the saccharide concentration and the simulated dilute solution fluorescent intensity, a mathematical function (or calibration curve) for determining the saccharide concentration is created, thus enabling the saccharide concentration of a dilute solution to be quantified from the fluorescent intensity of the dilute solution. Alternatively, the produced reaction product may be subjected to absorption analysis, with a calibration curve or the like created from the obtained absorbance values used to quantify the saccharide concentration. From the viewpoints of the detection limit and sensitivity, the use of fluorescence analysis is preferred.

The measurement method 2 includes quantifying the amount of the reaction product in the mixture following heating. The quantification may be conducted using any method typically used for quantifying a compound, and although the concentration of the saccharide may be quantified by using the type of fluorescence analysis or absorption analysis described for the measurement method 1, the quantification is not limited to these methods.

In yet another embodiment, the amount of saccharide in the case where the polysaccharide cell support dissolves completely in the cell suspension may be determined in advance. This means that based on the result of quantifying the concentration of saccharide, the proportion of the polysaccharide cell support in the cell suspension that has dissolved can be determined.

The measurement methods for the cell support-derived components in a liquid sample, wherein the cell support-derived components are derived from a polysaccharide cell support used in cell culture, described above enable measurement of the cell support-derived components in a liquid sample, wherein the cell support-derived components are derived from a polysaccharide cell support used in cell culture, to be conducted simply and with good sensitivity, and can be applied favorably to all manner of applications that utilize dissolvable cell supports.

### <Evaluation Methods> (not covered by the subject-matter of the claims)

Next is a description of methods for evaluating cell suspensions according to embodiments of the disclosure.

An evaluation method according to one embodiment is a method for evaluating a cell suspension containing cells that are cultured in the presence of a dissolvable cell support and collected following dissolution of the cell support, the method including providing an evaluation sample obtained from the cell suspension, preparing a mixture containing the evaluation sample, an acid and the specified aromatic compound, heating the mixture, and subjecting the reaction product in the mixture following heating to fluorescence analysis or absorption analysis (hereafter sometimes referred to as the evaluation method 1).

An evaluation method according to another embodiment is a method for evaluating a cell suspension containing cells that are cultured in the presence of a dissolvable cell support and collected following dissolution of the cell support, the method including providing an evaluation sample obtained from the cell suspension, preparing a mixture containing the evaluation sample, an acid, and the specified aromatic compound, heating the mixture, and quantifying the reaction product in the mixture following heating, wherein the mass of the aromatic compound is at least 200 times the mass of saccharides produced by hydrolysis of the cell support (hereafter sometimes referred to as the evaluation method 2).

**In** other words, the evaluation method 1 and the evaluation method 2 represent methods in which the measurement method 1 and the measurement method 2 respectively are applied to methods for evaluating cell suspensions. The evaluation method 1 and the evaluation method 2 are sometimes jointly referred to as simply "the evaluation method".

By using these evaluation methods, cell support-derived components in a cell suspension can be evaluated simply and with good sensitivity.

These evaluation methods include providing an evaluation sample obtained from a cell suspension, wherein this cell suspension contains cells that are cultured in the presence of a dissolvable cell support and collected following dissolution of the cell support.

In this description, the cells may be any adherent cells that can adhere to the cell support, and examples include somatic cells and stem cells and the like. Examples of the somatic cells include endothelial cells, epidermal cells, epithelial cells, myocardial cells, myoblasts, nerve cells, bone cells, osteoblasts, fibroblasts, adipose cells, hepatic cells, renal cells, pancreatic cells, adrenal gland cells, periodontal ligament cells, gingival cells, periosteal cells, skin cells, dendritic cells and macrophages.

The adherent cells are preferably animal-derived cells, and are more preferably mammalian-derived cells. Examples of the mammals include humans, monkeys, chimpanzees, cows, pigs, horses, sheep, goats, rabbits, rats, mice, marmots, dogs and cats. The adherent cells may be cells derived from tissues such as the skin, liver, kidneys, muscles, bone, blood vessels, blood, and nerve tissue.

The adherent cells may be of a single type of cell, or may be a combination of two or more types. In one embodiment, the adherent cells may be stem cells. Examples of the stem cells include somatic stem cells such as mesenchymal stem cells, hematopoietic stem cells, neural stem cells, myeloid stem cells and germline stem cells, and either mesenchymal stem cells or myeloid mesenchymal stem cells may be used. The term "mesenchymal stem cells" refers broadly to somatic stem cells which exist in various individual tissues, and can be differentiated into all or some mesenchymal cells such as osteoblasts, chondrocytes and adipocytes.

Pluripotent stem cells such as induced pluripotent stem cells (iPS cells), embryonic stem cells (ES cells), embryonic germ cells (EG cells), multipotent germline stem cells (mGS cells), embryonal carcinoma cells (EC cells), and Muse cells may also be used as the stem cells.

The medium used for culturing the cells preferably contains an inorganic salt, an amino acid, sugar and water. The medium may also contain optional components such as serum, vitamins, hormones, antibiotics, growth factors and adhesion factors. Media that are known as basal media for cell culture may be used as the medium.

In other words, any medium that is known for use in culturing the selected cells may be used as the medium without any particular limitations, and examples include, but are not limited to, DMEM (Dulbecco's Modified Eagle's Medium), MEM (Eagle's Minimum Essential Medium), and αMEM medium (α-Modified Eagle's Minimum Essential Medium).

In those cases where a serum is added to the medium, for example, serums such as fetal bovine serum (FBS), horse serum and human serum may be used.

The medium used for culturing may be free of xenogeneic components. Media that are free of xenogeneic components may include serum substitute additives (for example, Knockout Serum Replacement (KSR) (manufactured by Invitrogen Corporation), Chemically-defined Lipid Concentrate (manufactured by Gibco Inc.), and Glutamax (manufactured by Gibco Inc.) and the like) instead of animal-derived serum.

In addition, serum-free media such as Essential 8 (manufactured by Thermo Fisher Scientific Inc.), mTeSR1 (manufactured by STEMCELL Technologies Inc.), the StemFit series (manufactured by Takara Bio Inc.), and StemFlex (manufactured by Thermo Fisher Scientific Inc.).

Examples of dissolvable cell supports that can be used in the cell culturing include the same cell supports as those mentioned above in relation to the measurement methods. In terms of items relating to the dissolution of the cell support, those items described above may be applied, as is, to the evaluation methods.

By dissolving the cell support, the cells can be separated from the cell support. The cells that have been separated from the cell support can be collected using typically employed methods such as filtration using a filter, or methods that use centrifugal separation or the like. The collected cells may be suspended in an appropriate aqueous medium such a culture medium or physiological saline solution, enabling a cell suspension to be obtained.

**In** this description, the term "cell suspension" means a liquid that contains cells, and these cells may be in an adhered state or a non-adhered state to a cell support or a portion of a cell support.

The evaluation sample may be any sample that is obtained from the cell suspension, and for example, the cell suspension itself may be used as the evaluation sample, cells may be removed from the cell suspension and used as the evaluation sample, or a portion of the cell suspension or a portion of a liquid obtained by removing cells from the cell support may be mixed with another aqueous medium such as water to prepare the evaluation sample. The evaluation sample is preferably prepared using a liquid that does not contain cells. The evaluation sample may be prepared from the cell suspension immediately prior to evaluation, or a sample that has been prepared in advance as an evaluation sample may be procured.

The evaluation method 1 and the evaluation method 2 include preparing a mixture by mixing the evaluation sample prepared in the manner described above with an acid and the specified aromatic compound, and then executing the measurement method disclosed in this description, such as the measurement method 1 and the measurement method 2. In other words, the evaluation method 1 and the evaluation method 2 include preparing a mixture containing the evaluation sample prepared in the manner described above, an acid and at least one aromatic compound that can produce a fluorescent or light-absorbing compound as a result of a reaction with a saccharide under heating, and then using the obtained mixture to conduct the measurement method 1 and the measurement method 2 respectively.

In the evaluation method 1 and the evaluation method 2, the measurement method 1 and the measurement method 2 respectively are executed on the evaluation sample obtained from the cell suspension, and therefore the existence or absence of cell support-derived components in the evaluation sample, and by extension the cell suspension, can be confirmed simply and with good sensitivity, and in those cases where the cell suspension does contain cell support-derived components, the cell support-derived components within the cell suspension can be quantified simply and with good sensitivity.

Items described in relation to the measurement method 1 and the measurement method 2 in this description may also be applied to the evaluation method 1 and the evaluation method 2. Items described in this description in relation to the measurement method, without any limitation to the measurement method 1 and the measurement method 2, may also be applied to the evaluation method 1 and the evaluation method 2. Items described in this description in relation to the evaluation method, without any limitation to the evaluation method 1 and the evaluation method 2, may also be applied to the measurement method 1 and the measurement method 2. Items described in this description in relation to the measurement method, without any limitation to the measurement method 1 and the measurement method 2, may also be applied to the evaluation method, without any limitation to the evaluation method 1 and the evaluation method 2.

The target for the measurement method and evaluation method according to the first embodiment is not limited to cell support-derived components within the cell suspension, and may also be a saccharide component not derived from the cell support. For example, the measurement method and the evaluation method according to the first embodiment may be used for quantifying glucose in a cell suspension following preparation, in the case where the cell suspension is prepared by collecting cells following culturing, removing the medium components, and then suspending the cells in a buffer such as PBS. This enables the cell suspension to be evaluated for residual medium components in the cell suspension following removal of the medium components.

### (Second Embodiment)

One method of cell culturing involves using microcarriers to achieve three-dimensional culturing of cells. Microcarriers are small particles composed of a dissolvable or indissolvable polymer that mainly adopt a spherical shape, and can be used to support and promote the growth of adherent cells in a cell suspension such as a culture medium used for cell culture. Because cell culturing using microcarriers is a three-dimensional form of cell culture, large-scale culturing of the cells is possible, and the floor area required for the cell culture can be reduced. Following the cell culture, the cells can be detached from the microcarriers using an enzyme or the like and the microcarriers removed, enabling the cells to be collected.

Cell suspensions are sometimes prepared by suspending cells that have been separated from microcarriers in another medium or liquid, and the resulting cell suspension sometimes contains microcarrier fragments smaller than a prescribed size as impurities. These types of impurities include, for example, fragments and the like that may be incorporated in the microcarrier raw materials. Accordingly, with cell culturing using microcarriers, it is desirable to be able to evaluate whether or not impurities derived from the microcarriers exist in the resulting cell suspension. In the case of large-scale cell culturing, precise control tends to become difficult, and therefore evaluation of the presence or absence of contaminated with impurities is important.

### <Cell Suspension Evaluation Method>

A method for evaluating a cell suspension according to one embodiment includes adding a fluorescent dye that is capable of staining a polymer having a hydrophobic portion to a cell suspension containing cells, irradiating the cell suspension containing the added fluorescent dye with an excitation light and performing fluorescence analysis, and then evaluating the cell suspension based on the fluorescence analysis.

By using this method, a cell suspension can be provided which has been evaluated for the presence or absence of contamination with impurities derived from the microcarriers. For example, a cell suspension can be provided which has been evaluated for the presence or absence of contamination with a polymer having a hydrophobic portion derived from the microcarriers. In one embodiment, when providing a cell suspension, the cell suspension can be provided with information that details the existence and amount and the like of the contamination by impurities derived from the microcarriers.

The cell suspension containing cells is a liquid in a state in which the cells are dispersed within a dispersion medium. The dispersion medium for the cell suspension is preferably an aqueous liquid, is more preferably a medium containing water as the main solvent, and may be, for example, a buffer solution such as a phosphate buffer, a culture medium, a physiological saline solution, or pure water or the like.

The cell suspension that functions as the evaluation target may be a suspension obtained following cell culturing, or a cell suspension prior to cell culturing. Further, the cell suspension of the evaluation target may also contain a cell support such as microcarriers, or may have had a cell support such as microcarriers added and subsequently removed. Furthermore, the cell suspension of the evaluation target may also contain typical additives used in cell culture.

There are no particular limitations on the concentration of the cells in the cell suspension of the evaluation target. In those cases where a polymer having a hydrophobic portion is measured within a cell suspension of high concentration, an arbitrary liquid may first be added to adjust the concentration of the cell suspension before the fluorescent dye is added.

The fluorescent dye that is added to the cell suspension for the purpose of evaluating the cell suspension is preferably a fluorescent dye that is capable of staining a polymer having a hydrophobic portion.

Examples of such fluorescent dyes that are capable of staining a polymer having a hydrophobic portion include compounds containing a chromophore having an aromatic ring. The chromophore may have, for example, a monocyclic ring such as a benzene ring, non-benzene ring or heteroaromatic ring; a condensed aromatic ring in which one type or two or more types of monocyclic ring have been condensed; or a polycyclic structure in which a plurality of these monocyclic rings, condensed aromatic rings, or a combination thereof have been bonded together via arbitrary bonding. In order to ensure affinity with the polymer having a hydrophobic portion to facilitate staining, the chromophore may be a polycyclic structure having two or more monocyclic rings, a polycyclic structure containing a condensed aromatic ring structure with two or more rings, and preferably three or more rings, or a polycyclic structure containing a total of two or more monocyclic rings and condensed aromatic rings.

Examples of the benzene ring, non-benzene ring, or condensed aromatic ring thereof include a benzene ring, cyclopentadiene ring, naphthalene ring, anthracene ring, phenanthrene ring, tetracene ring, triphenylene ring, chrysene ring, pyrene ring, perylene ring, perinone ring, and fluorene ring.

The heteroaromatic ring preferably has at least one atom selected from the group consisting of N, O, P and S within the ring structure, and is more preferably a heteroaromatic ring having N, O or a combination thereof within the ring structure.

Examples of the heteroaromatic ring or condensed heteroaromatic ring include a pyrrole ring, pyridine ring, imidazole ring, pyrazole ring, oxazole ring, thiazole ring, pyrazine ring, pyrimidine ring, indole ring, benzimidazole ring, quinoline ring, isoquinoline ring, acridine ring, carbazole ring, furan ring, pyran ring, benzofuran ring, dibenzofuran ring, benzopyran ring, xanthene ring, fluorescein ring, thiophene ring, benzothiophene ring, dibenzothiophene ring, porphyrin ring, dipyrromethene ring, and phthalocyanine ring.

Among the various fluorescent dyes, those that readily dye polymers having a hydrophobic portion but are unlikely to stain, or do not stain, the cells are preferred. Examples of such fluorescent dyes include low-molecular weight compounds having two or more monocyclic aromatic rings, a condensed aromatic ring, or a combination of a monocyclic aromatic ring and a condensed aromatic ring; and high-molecular weight compounds having two or more monocyclic aromatic rings, a condensed aromatic ring, or a combination of a monocyclic aromatic ring and a condensed aromatic ring. Specific examples include porphyrin, phthalocyanine, poly(phenylene vinylene), pyrene, perylene, perinone, xanthene, naphthofluorescein, and derivatives of these compounds.

A hydrophilic group may be introduced into the chromophore of the fluorescent dye or a region near the chromophore. Examples of the hydrophilic group include anionic groups, cationic groups, an amino group, a hydroxyl group, polyalkylene glycol groups, and a carboxyl group. Examples of the polyalkylene glycol groups include a polyethylene glycol group, polypropylene glycol group, and polyethylene-propylene glycol group. The number of added moles in the polyalkylene glycol group is, for example, within a range from 2 to 2,000, and is preferably from 2 to 200. By including a hydrophilic group in the fluorescent dye, the solubility in the dispersion medium can be improved.

An ionic group may be introduced into the chromophore of the fluorescent dye or a region near the chromophore. The ionic group may be either an anionic group or a cationic group. Because microcarriers have often undergone a cation treatment from the viewpoint of cell adhesion, by introducing an anionic group into the fluorescent dye, the fluorescent dye can more effectively bond with and stain the polymer having a hydrophobic portion derived from the microcarriers. Examples of the anionic group include a sulfonate group, phosphate group, carboxyl group, and nitrate group. From the viewpoint of cell compatibility, a sulfonate group can be used particularly favorably.

The fluorescent dye capable of staining a polymer having a hydrophobic portion is not limited to the above compounds, and low-molecular weight compounds containing a chromophore having an aromatic ring may also be used, regardless of whether or not they stain cells. Examples of these types of compounds include compounds containing one or two or more, and preferably one or two, monocyclic rings, two-ring condensed aromatic rings, or three-ring condensed aromatic rings. Specific examples include coumarin dyes, DCM dyes, cyanine dyes, quinoline dyes, and dipyrromethene dyes.

Specific examples of the fluorescent dye capable of staining a polymer having a hydrophobic portion include porphyrin dyes; phthalocyanine dyes; high-molecular weight compounds such as poly(phenylene vinylene) or derivatives thereof; compounds having a condensed aromatic ring structure such as pyrene, perylene and perinone; xanthene dyes such as fluorescein and naphthofluorescein; triarylmethane dyes such as triphenylmethane; coumarin dyes such as coumarin, coumarin 6 and coumarin 30; DCM dyes such as 4-(dicyanomethylene)-2-methyl-6-(4-dimethylaminostyryl)-4H-pyran, 2-tert-butyl-4-(dicyanomethylene)-6-[2-(1,1,7,7-tetramethyljulolidin-9-yl)vinyl]-4H-pyran, and 4-(dicyanomethylene)-2-methyl-6-[2-(2,3,6,7-tetrahydro-1H,5H-benzo[ij]quinolizin-9-yl)vinyl]-4H-pyran; cyanine dyes; quinoline dyes; and dipyrromethene dyes such as BODIPY (a registered trademark, boron-dipyrromethene).

One of these dyes may be used alone, or a combination of two or more dyes may be used, provided the effects of the disclosure are not impaired.

At least one dye selected from the group consisting of porphyrin dyes, phthalocyanine dyes, poly(phenylene vinylene) dyes, pyrene dyes, xanthene dyes, coumarin dyes and DCM dyes is preferred.

Here, the term "porphyrin dyes" means dyes containing porphyrin or a derivative thereof, and this naming convention also applies to the naming of the other dyes.

Among the fluorescent dyes described above, polyvalent anion-based compounds, compounds having a polyalkylene glycol chain, naphthofluorescein, coumarin dyes and DCM dyes and the like can be used favorably, and in terms of enabling selective staining of polymers having a hydrophobic portion within the cell suspension, the use of polyvalent anion-based compounds, compounds having a polyalkylene glycol chain and naphthofluorescein is particularly preferred, and in terms of suppressing staining of cells within the cell suspension while enabling staining of polymers having a hydrophobic portion, the use of a polyvalent anion-based compound is even more desirable.

The polyvalent anion-based compound may be a compound having two or more anionic groups introduced into the chromophore, and compounds having four or more anionic groups introduced into the chromophore are more preferred. For example, compounds having two or more anionic groups introduced into a low-molecular weight compound having a condensed aromatic ring can stain polymers having a hydrophobic portion selectively in preference to the cells. Examples of these types of compounds include polyvalent anions of porphyrin, phthalocyanine or derivatives thereof. A specific example is 5,10,15,20-tetrakis(4-sulfophenyl)porphyrin (TPPS).

Further examples include compounds having two or more anionic groups introduced into a high-molecular weight compound having a monocyclic ring or a condensed aromatic ring. Examples include compounds having two or more anionic groups introduced into copolymers containing a plurality of structural units having an aromatic ring, and copolymers containing a plurality of structural units having an aromatic ring and an anionic group. Specific examples of such compounds include polyvalent anions of poly(phenylene vinylene) or derivatives thereof. The molecular weight of these high-molecular weight compounds is, for example, typically within a range from 100 to 100,000, and is preferably from 100 to 10,000. Specific examples include poly(5-methoxy-2-(3-sulfopropoxy)-1,4-phenylene vinylene) and the like.

The compounds having a polyalkylene glycol chain may be compounds having a polyalkylene glycol chain introduced into the chromophore. The chromophore is preferably a condensed benzene ring structure such as pyrene, perylene or perinone. Examples of the polyalkylene glycol chain include polyethylene glycol chains, polypropylene glycol chains, and polyethylene-propylene glycol chains. The number of added moles in the polyalkylene glycol chain is, for example, within a range from 2 to 2,000, and is preferably from 2 to 200. Because polyalkylene glycol chains have hydrophilicity, polymers having a hydrophobic portion can be more easily stained with the fluorescent dye. Specific examples include methoxypolyethylene glycol pyrene and the like.

Naphthofluorescein is a fluorescent dye having hydroxyl groups introduced into or near a chromophore having a xanthene structure, and has a property that the fluorescent intensity varies depending on the pH of the system.

The fluorescent dye may, for example, have a property wherein the fluorescent characteristics differ between intracellular environments and extracellular environments. In such cases, when the fluorescent dye exists in a cell suspension, a differentiation can be made between the fluorescence emitted by cells having the fluorescent dye incorporated therein and the fluorescence emitted by polymers having a hydrophobic portion, thus enabling the existence of a polymer having a hydrophobic portion within the cell suspension to be confirmed.

For example, in the case of a fluorescent dye that does not emit fluorescence in intracellular environments, but does emit fluorescence in extracellular environments, only the fluorescence from polymers having a hydrophobic portion that exist in the extracellular environment can be detected. As a result, the existence of a polymer having a hydrophobic portion can be confirmed. In another example, in the case of a fluorescent dye for which the fluorescent intensity emitted in intracellular environments is low, but the fluorescent intensity emitted in extracellular environments is high, the difference in fluorescent intensity in the cell suspension can be used to distinguish the existence of a polymer having a hydrophobic portion from the cells.

Fluorescent dyes for which the wavelength of the excitation light that causes fluorescence differs between intracellular and extracellular environments may also be used. For example, by utilizing the fact that the fluorescent intensity of the fluorescent dye in an intracellular environment is increased by excitation light within a first wavelength range, whereas the fluorescent intensity of the fluorescent dye in an intracellular environment is lowered by excitation light within a second wavelength range, polymers having a hydrophobic portion can be distinguished from cells and identified by using excitation light within the second wavelength range.

Fluorescent dyes for which the hue of the fluorescence emitted in response to the excitation light differs between intracellular and extracellular environments may also be used.

Examples of this type of fluorescent dye, from among the various fluorescent dyes described above, include compounds having a polyalkylene glycol chain and naphthofluorescein.

Compounds having a polyalkylene glycol chain exhibit a difference in the ease of multimer formation between intracellular and extracellular environments, with multimers tending to form more readily, leading to increased fluorescent intensity, in extracellular environments.

In those case where a localized difference in pH between intracellular and extracellular environments is to be utilized, naphthofluorescein can be used, which exhibits a variation in fluorescent intensity depending on the pH.

As one example of the fluorescent dye, it is preferable that in a cell suspension, the fluorescent intensity emitted as a result of staining of a polymer having a hydrophobic portion with the fluorescent dye is higher than the fluorescent intensity emitted as a result of staining of the cells with the fluorescent dye. Confirmation that, within the cell suspension, the fluorescent intensity of a polymer having a hydrophobic portion is higher than the fluorescent intensity of the cells can be made by observing or measuring the intensity of the fluorescence. In one embodiment, when excitation light of a prescribed wavelength is irradiated, no cell fluorescence is detected in the cell suspension, but fluorescence from a polymer having a hydrophobic portion can be detected. For example, upon irradiation of excitation light of a prescribed wavelength, the fluorescent intensity of the cells in the cell suspension is less than a certain fixed value (a), whereas the fluorescent intensity of the polymer having a hydrophobic portion is at least as high as the fixed value (a), or is at least 1.1 times the fixed value (a), or preferably at least 2 times the fixed value (a).

Depending on the type of fluorescent dye, the wavelength of the irradiated excitation light differs, but an excitation light having a wavelength range that yields stronger fluorescence from the fluorescent dye is preferably selected.

Examples of such fluorescent dyes include the polyvalent anion-based compounds mentioned among the fluorescent dyes described above.

Another example of the fluorescent dye is a fluorescent dye having a characteristic wherein, in a cell suspension, when at least one property selected from the group consisting of the temperature, concentration and pH of the cell suspension satisfies a certain range, the fluorescent intensity emitted as a result of staining of a polymer having a hydrophobic portion with the fluorescent dye is either higher or lower than the fluorescent intensity emitted as a result of staining of the cells with the fluorescent dye. When this type of fluorescent dye is used, by detecting the fluorescent intensity after appropriate adjustment of the temperature or the like of the cell suspension, the cells and the polymer having a hydrophobic portion can be easily distinguished.

For example, when a fluorescent dye is used for which fluorescence is detected depending on whether the pH conditions are acidic or alkaline, the fluorescent dye is added to the cell suspension, and the fluorescence can then be detected following adjustment of the cell suspension to an acidic or alkaline state. A similar process applies for other conditions such a temperature and concentration, and combinations of these conditions may also be employed.

Depending on the type of fluorescent dye, the wavelength of the irradiated excitation light differs, but irradiation with an excitation light having a wavelength range that yields a strong fluorescence from the fluorescent dye is preferred.

Examples of fluorescent dyes for which the fluorescent intensity can be adjusted by altering the concentration conditions include the compounds having a polyalkylene glycol chain mentioned among the fluorescent dyes described above. Examples of fluorescent dyes for which the fluorescent intensity can be adjusted by altering the pH conditions include naphthofluorescein and the like from among the fluorescent dyes described above.

There are no particular limitations on the method used for adding the fluorescent dye to the cell suspension, and for example, a solid fluorescent dye in powder form or granular form or the like may be added to the cell suspension, or a composition prepared by dispersing or dissolving the fluorescent dye in a solvent may be added to the cell suspension.

Following addition of the fluorescent dye to the cell suspension, the resulting mixture is preferably mixed or stirred to ensure uniform blending of the fluorescent dye with the cell suspension.

The polymer having a hydrophobic portion is a component that can exist in the cell suspension during cell culture using microcarriers. The cell suspension obtained by removing the microcarriers following cell culturing contains cells and a liquid, and it is preferable that the amount of solids other than the cells is small, meaning, for example, that the amount of the polymer having a hydrophobic portion is preferably small. Because the microcarriers are typically of a larger size than the cells, following the cell culture, the microcarriers can be removed from the cell suspension, for example, using a filter or the like. However, in the case of microcarrier fragments of a size smaller than the cells, these fragments may sometimes not be removed from the cell suspension by filtering or the like. In other words, the polymers having a hydrophobic portion that may exist in the cell suspension may include microcarrier materials. Further, in the case of microcarriers that can be dissolved by enzymes or the like, dissolution residues having a size smaller than the cells may exist in the cell suspension. In those cases where these dissolution residues include polymers having a hydrophobic portion, these dissolution residues can also be detected.

Microcarriers are described below in further detail.

The material for the microcarriers may be an organic substance, an inorganic substance or a composite material thereof. Of these materials, in those cases where cell culturing is conducted using microcarriers formed using a polymer having a hydrophobic portion as the organic substance, the method of one embodiment of the disclosure is useful for evaluating the polymer having a hydrophobic portion in the cell suspension.

Examples of the organic substances include synthetic polymers such as polystyrene, polyester, polyurethane, polyethylene, polypropylene, (meth)acrylic-based polymers, (meth)acrylamide-based polymers, polyvinyl alcohol, silicone-based polymers and epoxy resins; natural polymers such as collagen and gelatin; polygalacturonic acids such as pectin and pectates; and polysaccharides such as alginates, cellulose, crosslinked agarose, dextran and chitosan. Examples of the inorganic substances include glass, ceramics, metals, alloys and metal oxides.

From the viewpoint of cytocompatibility, the microcarriers preferably contain an organic substance. **In** one embodiment, in those cases where the microcarriers are formed using a polymer having a hydrophobic portion, the polymer having a hydrophobic portion that may contaminate the cell suspension can be evaluated. Examples of the polymer having a hydrophobic portion include those polymers among the above synthetic polymers and natural polymers that exhibit hydrophobicity, or polymers obtained by subjecting the synthetic polymers and natural polymers described above to a hydrophobic treatment. Typical examples include polystyrene and polyester and the like, and polystyrene is preferred. Further, polymers obtained by introducing hydrophobic groups into polyvinyl alcohol, sodium salts of (meth)acrylic-based polymers, or hydrophilic polymers such as copolymers having a large amount of introduced hydrophilic groups may also be used as microcarriers. Typical examples of the hydrophobic groups include chain-like or cyclic hydrocarbon groups and aromatic hydrocarbon groups and the like.

From the viewpoint of cytocompatibility, microcarriers formed from a hydrogel may also be used. Examples of hydrogels that may be used include those obtained by introducing hydrophobic groups into polyvinyl alcohol, sodium salts of (meth)acrylic-based polymers, or hydrophilic polymers such as gelatin or copolymers having a large amount of introduced hydrophilic groups. Typical examples of the hydrophobic groups include chain-like or cyclic hydrocarbon groups and aromatic hydrocarbon groups and the like.

A single type of microcarrier may be used alone, or a combination of two or more microcarriers may be used. Among the various options, hydrophobic polymers or polymers having a hydrophobic group can function as the evaluation target polymer in one embodiment, and can therefore be used favorably.

A polymer having a hydrophobic portion contained in the cell suspension can be used as an evaluation target even in those cases were the polymer is not derived from microcarriers.

From the viewpoint of promoting cell adhesion, cationic functional groups may be introduced onto the surface of the microcarriers. Examples of the cationic functional groups include groups containing substituted or unsubstituted cationic amino groups such as dimethylamino groups, diethylamino groups and amino groups.

From the viewpoint of promoting cell adhesion, a cell-adhesive polymer may be disposed at the surface of the microcarriers. Examples of the cell-adhesive polymer include collagen, gelatin, alginic acid, Matrigel (a registered trademark) (manufactured by BD Biosciences Ltd.), hyaluronic acid, laminin, fibronectin, vitronectin, elastin, heparan sulfate, dextran, dextran sulfate and chondroitin sulfate.

Examples of the shape of the microcarriers include spherical shapes, flat shapes, cylindrical shapes, plate shapes and prismatic shapes. The microcarriers preferably include a spherical microcarrier. The microcarriers may be a porous microcarrier having internal pores or microcarriers that has no internal pores.

From the viewpoint of promoting cell proliferation, the average particle size (D50) of the microcarriers are, for example, within a range from 50 to 1,000 µm, and is preferably from 100 to 500 µm, more preferably from 120 to 250 µm, and even more preferably from 150 to 250 µm. The average particle size of the microcarriers are the value measured for the median size (D50) in a physiological saline solution or in a culture medium. The average particle size of the microcarriers can be measured using a laser diffraction/scattering-type particle size distribution analyzer.

The microcarriers used in cell culturing are preferably larger than the size of the cells supplied to the cell culture.

A method for evaluating a cell suspension by adding a fluorescent dye to the cell suspension, irradiating the suspension with an excitation light, and then analyzing the fluorescence is described below.

There are no particular limitations on the method used for analyzing the fluorescence of the cell suspension containing the added fluorescent dye.

In those cases where an excitation light is irradiated onto the cell suspension containing the added fluorescent dye, the existence of a polymer having a hydrophobic portion within the cell suspension can be confirmed by observing the fluorescence in the cell suspension. In order to enable confirmation of whether or not a polymer having a hydrophobic portion exists in the cell suspension, it is preferable that, within the cell suspension, either the cells do not fluoresce whereas the polymer having a hydrophobic portion does fluoresce, or the fluorescence from the cells is weak whereas the fluorescence from the polymer having a hydrophobic portion is strong. Examples of fluorescent dyes that can be used in such situations include the polyvalent ionic compounds from among the fluorescent dyes described above. Further, a fluorescent dye for which the florescence characteristics differ depending on the cell suspension environment may also be used, and examples of fluorescent dyes that can be used in such situations include the compounds having a polyalkylene chain and naphthofluorescein mentioned among the fluorescent dyes described above.

Furthermore, by analyzing the fluorescence from the cell suspension, differences in the fluorescent intensity can be used to contrast and evaluate the amounts of polymers having a hydrophobic portion.

By analyzing the fluorescence from the cell suspension, the existence of a polymer having a hydrophobic portion or the amount of that polymer can be evaluated, but the size, shape, or concentration or the like of the polymer having a hydrophobic portion, or a combination of these properties, may also be measured. For example, by irradiating an excitation light onto the cell suspension containing the added fluorescent dye while observing the cell suspension with a fluorescence microscope, in those cases where the cell suspension contains a polymer having a hydrophobic portion, the stained polymer having a hydrophobic portion that has been stained by the fluorescent dye can be observed. Further, by conducting image analysis of the image obtained from the fluorescence microscope observation, the size, distribution, and concentration and the like of the polymer having a hydrophobic portion can be measured. In another method, by irradiating an excitation light onto the cell suspension containing the added fluorescent dye while conducting spectrofluorophotometric analysis, in those cases where the cell suspension contains a polymer having a hydrophobic portion, the fluorescent intensity relative to a blank can be confirmed. Moreover, by producing a calibration curve in advance, the concentration and the like of the polymer having a hydrophobic portion can be measured based on the fluorescent intensity.

The excitation light irradiated onto the cell suspension preferably has a wavelength range that is selected in accordance with the type of fluorescent dye to ensure a strong emission of fluorescence.

The method for evaluating a cell suspension may include evaluating the existence in the cell suspension of a polymer having a hydrophobic portion with a particle size equal to or smaller than the cell size.

One method that may be used for removing only the cells from the cell suspension following cell culture involves using a filter or the like to remove solid matter other than cells from the cell suspension, based on the difference in size between the cells and the solid matter other than cells. This method is suitable for removing solid matter having a larger size than the cells, but is not suitable for removing solid matter that is either smaller than the cells, or of a similar size to the cells. Solid matter having a size much smaller than the cells can be removed by washing, using a method such as substitution of the cell suspension solvent. On the other hand, if polymer fragments and the like derived from the microcarriers raw material supplied during cell culture are of a similar size to, or smaller than, the cells, then there is a possibility that these fragments of small particle size will be retained and not removed from the cell suspension.

In one embodiment, the evaluation method described above can be used to evaluated whether or not a cell suspension contains a polymer having a hydrophobic portion of small particle size. For example, the existence of a polymer having a hydrophobic portion with a small particle size in the cell suspension can be evaluated by adding a fluorescent dye to the cell suspension and detecting the fluorescence. Further, as described above in relation to the evaluation method, by observing the cell suspension containing the added fluorescent dye with a fluorescence microscope, the existence or absence of a polymer having a hydrophobic portion, and the shape, distribution and concentration and the like of the polymer having a hydrophobic portion can be evaluated. Furthermore, analyzing the cell suspension containing the added fluorescent dye by spectrofluorophotometric analysis, in those cases where the cell suspension contains a polymer having a hydrophobic portion, the existence or absence of a polymer having a hydrophobic portion can be evaluated, and by creating a calibration curve, the concentration and the like of the polymer having a hydrophobic portion can also be evaluated.

In this description, the size of the polymer having a hydrophobic portion can be determined from a photographic image obtained from the fluorescence microscope observation. For example, the small-fragment polymer having a hydrophobic portion that is suitable as an evaluation target may have a particle size within a range from 100 nm to 100 µm, or within a range from 100 nm to 10 µm.

In the disclosure, the size of the cells may vary depending on the type of cell, but the size is typically not more than 100 µm, and may be not more than 60 µm, not more than 30 µm, or 20 µm or smaller. Further, although there are no particular limitations on the cell size, the cells are typically at least 1 µm, and may be at least 5 µm, or 10 µm or larger. For example, the cell suspension may contain cells of 1 to 100 µm, and preferably contains cells of 5 to 20 µm.

There are no particular limitations on the method used for measuring the size of the cells, and for example, the size may be measured by a conventional method using a cell sorter or a flow cytometer.

The method for evaluating a cell suspension may include evaluating the existence of a polymer having a hydrophobic portion and having a particle size 0.01 times to 5 times the size of the cells in the cell suspension.

The particle size of the polymer having a hydrophobic portion that represents the evaluation target is preferably at least 0.01 times the size of the cells, and may be at least 0.05 times, or at least 0.1 times, the size of the cells. By ensuring that the particle size of the polymer having a hydrophobic portion falls within this range, fluorescence can be detected when the fluorescent dye stains the polymer having a hydrophobic portion and emits fluorescence.

The particle size of the polymer having a hydrophobic portion that represents the evaluation target is preferably not more than 5 times the size of the cells, and may be not more than 4 times, or not more than 3 times, the size of the cells. A polymer having a hydrophobic portion with a particle size within this range may be contained in the cell suspension even in the state following microcarrier removal following cell culture, washing, or a combination thereof, and therefore can function as an evaluation target.

For example, the particle size of the polymer having a hydrophobic portion that represents the evaluation target may be within a range from 0.01 times to 5 times the size of the cells, and may be within a range from 0.05 times to 4 times the size of the cells, or from 0.1 times to 3 times the size of the cells.

The cell suspension may be a cell suspension obtained by conducting cell culturing using cells and microcarriers, and then separating the cells and the microcarriers following cell culture to remove the microcarriers. The fluorescent dye added to this cell suspension obtained following cell culture preferably stains polymers having a hydrophobic portion that are of the same material as the microcarriers used in the cell culture. For example, the types of fluorescent dyes described above may be used.

Accordingly, polymers having a hydrophobic portion and with a small particle size, such as microcarrier fragments that may be incorporated in the cell suspension, can be evaluated in a state following removal of the microcarriers used in the cell culture from the cell suspension following the cell culture.

There are no particular limitations on the method used for removing the microcarriers from the cell suspension following cell culture, and the microcarriers may be detached from the cells using an appropriate method, with the difference in size between the microcarriers and the cells utilized to remove the larger particle size microcarrier using a filter or the like.

Washing of the cell suspension following the cell culture may be included as an optional step. This enables the removal of solid matter in the cell suspension that is smaller than the cells, such as small fragments that may be incorporated within the microcarrier raw materials.

**In** those case where a cell suspension following cell culture is used, it is preferable that at least one of two evaluations is conducted, namely (1) an evaluation of the existence of a polymer having a hydrophobic portion with a particle size equal to or smaller than the size of the cells in the cell suspension, and (2) an evaluation of a polymer having a hydrophobic portion with a particle size equal to or smaller than the average particle size of the microcarriers used in the cell culture. In those cases where the evaluation (1) and the evaluation (2) are combined, the evaluation (2) may be performed after the evaluation (1), the opposite sequence may be used, or the evaluation (1) and the evaluation (2) may be performed simultaneously.

As a result, polymers having a hydrophobic portion with a small particle size derived from the microcarriers, which may be contained in the cell suspension, can be measured following removal of the microcarriers that were supplied to the cell culture from the cell suspension following the cell culture.

As described above, following the cell culture, solid matter of a size larger than the cells can be removed from the cell suspension using a filter or the like, but in some cases, solid matter that is larger than the cells, but of a size smaller than the hydrophobic microcarrier used in the cell culture, may be retained in the cell suspension.

Accordingly, evaluation may be conducted not only of the existence of polymers having a hydrophobic portion with a particle size that is equal to or smaller than the size of the cells, but also of the existence of polymers having a hydrophobic portion with a particle size that is larger than the size of the cells but equal to or smaller than the average particle size of the microcarriers. For example, in those cases where a filter is used that has a pore size suited to the removal of the microcarriers from the cell suspension, polymers having a hydrophobic portion with a particle size that smaller than the average particle size of the microcarriers will be incorporated in the cell suspension. Examples of these polymers having a hydrophobic portion with a small particle size include small fragments derived from microcarrier raw materials.

### <Cell Suspension>

The cell suspension according to one embodiment is characterized by containing cells, wherein the number of polymers having a hydrophobic portion and a size of 1 µm or smaller is 10 or fewer per 1×10⁴ cells.

This enables a cell suspension to be provided that has been evaluated for the existence or absence of contamination with impurities derived from the microcarriers.

The cell suspension is a liquid that exists in a state where cells are dispersed within a dispersion medium. Details are as described above. The liquid provided as a cell suspension has preferably undergone removal of the cell support such as microcarriers following the cell culture.

The cell suspension from which the cell support such as microcarriers has been removed following cell culture preferably contains none of the microcarriers themselves, and preferably also contains no portions of the microcarriers, such as polymers having a hydrophobic portion with a small particle size derived from the hydrophobic microcarrier.

In the cell suspension, the number of polymers having a hydrophobic portion and a size that is smaller than a typical cell is preferably small, and for example, the number of polymers having a hydrophobic portion with a size of 1 µm or smaller is preferably small.

In the cell suspension, the number of polymers having a hydrophobic portion with a size of 1 µm or smaller per 1×10⁴ cells is preferably 10 or fewer, more preferably 8 or fewer, and even more preferably 6 or fewer.

In the cell suspension, the number of polymers having a hydrophobic portion with a size of 1 µm or smaller per 1×10⁴ cells may be zero, namely, may not be detected at all, but the number per 1×10⁴ cells may exceed zero, and may be 1 or more, in those cases where no additional procedures are required to completely remove these polymers having a hydrophobic portion of small particle size.

For example, the number of polymers having a hydrophobic portion with a size of 1 µm or smaller, per 1×10⁴ cells, may be within a range from 0 to 10, from greater than 0 to 8, or from 1 to 6.

Here, in the cell suspension, the method for measuring the number of polymers having a hydrophobic portion with a size of 1 µm or smaller per 1×10⁴ cells may be conducted by adding a fluorescent dye to the cell suspension to stain the polymers having a hydrophobic portion, subsequently conducting image observation using a microscope or the like, measuring the particle size of polymers having a hydrophobic portion observed within a prescribed region, and then counting the number of cells and the number of polymers having a hydrophobic portion with a size of 1 µm or smaller observed within that prescribed region. Further, the sizes of polymers having a hydrophobic portion may also be determined from a photographic image acquired by fluorescence microscope observation.

The number of polymers having a hydrophobic portion with a size of 1 µm or smaller per 1×10⁴ cells can also be determined by image analysis by arithmetic processing using the image used in image observation. The particle sizes of these polymers having a hydrophobic portion may also be determined by image analysis in a similar manner.

In the image observation, the cell suspension may be used without modification, or a solvent may be added to dilute the cell suspension, provided the ratio between the cells and the polymers having a hydrophobic portion is not altered, with this diluted cell suspension then used for the observation.

The cell suspension according to another embodiment contains cells and a fluorescent dye that is capable of staining a polymer having a hydrophobic portion, wherein the fluorescent intensity emitted due to staining of the polymer having a hydrophobic portion by the fluorescent dye is higher than the fluorescent intensity emitted caused by staining of the cells by the fluorescent dye.

This enables a cell suspension to be provided that has been evaluated for the existence or absence of contamination with impurities derived from the microcarriers.

The cell suspension is a liquid that exists in a state where cells are dispersed within a dispersion medium. Details are as described above. The liquid provided as a cell suspension has preferably undergone removal of the cell support such as microcarriers following the cell culture.

The cell suspension from which the cell support such as microcarriers has been removed following cell culture preferably contains none of microcarriers themselves, and preferably also contains no portions of the microcarriers, such as polymers having a hydrophobic portion with a small particle size derived from the hydrophobic microcarrier.

For example, as described above, in the cell suspension, the number of polymers having a hydrophobic portion with a size of 1 µm or smaller is preferably 10 or fewer per 1×10⁴ cells.

This cell suspension preferably contains a fluorescent dye as well as the cells. The fluorescent dye is as described above. When the cell suspension containing the cells and a fluorescent dye is irradiated with an excitation light, fluorescence can be detected in those cases where the cell suspension contains a polymer having a hydrophobic portion. In other words, when the cell suspension containing the cells and a fluorescent dye is irradiated with an excitation light, no fluorescence should be detected in those cases where either the cell suspension does not contain a polymer having a hydrophobic portion, or the amount of the polymer having a hydrophobic portion is small enough that no fluorescence is detected.

For example, in the case of this cell suspension, the fluorescent dye may be added after the cell culture, and by irradiating the cell suspension with the excitation light at the time of use, the existence of a polymer having a hydrophobic portion within the cell suspension can be detected, or the concentration or the like of the polymer can be confirmed from the fluorescent intensity. In those cases where the step of preparing the cell suspension and the step of using the cell suspension are separated either in terms of time or spatial separation, even if strict product control is not conducted during the step of preparing the cell suspension, the existence of a polymer having a hydrophobic portion within the cell suspension, or the concentration or the like of the polymer based on the fluorescent intensity, can be easily confirmed during the step of using the cell suspension.

### <Reagent for Evaluating Cell Suspension>

A reagent for evaluating a cell suspension according to one embodiment is characterized by containing a fluorescent dye that is capable of staining a polymer having a hydrophobic portion and a buffer solution, wherein the osmotic pressure is within a range from 100 to 400 mosm/Kg.

By using this reagent, the existence or absence of contamination of the cell suspension with microcarrier-derived impurities can be evaluated.

By adding this reagent to a cell suspension, in those cases where the cell suspension contains a polymer having a hydrophobic portion, the existence of that polymer having a hydrophobic portion in the cell suspension can be evaluated by irradiating an excitation light onto the cell suspension and detecting the fluorescence from the cell suspension. In those cases where the cell suspension contains no polymers having a hydrophobic portion, no fluorescence should be detected from the cell suspension.

By adding this reagent to a cell suspension, irradiating the cell suspension with an excitation light, and observing the fluorescence from the cell suspension with a fluorescence microscope, if a polymer having a hydrophobic portion exists in the cell suspension, then the shape, distribution and concentration and the like of the polymer having a hydrophobic portion can be evaluated.

Further, by adding this reagent to a cell suspension, irradiating the cell suspension with an excitation light, and analyzing the fluorescence from the cell suspension by spectrofluorophotometric analysis, if a polymer having a hydrophobic portion exists in the cell suspension, then the amount and the concentration and the like of the polymer having a hydrophobic portion can also be evaluated.

Details of these processes are as described in the procedures described above.

In this reagent, the types of fluorescent dyes described above can be used for the fluorescent dye. A phosphate buffer solution or the like can be used as the buffer solution in this reagent. The polymer having a hydrophobic portion that represents the evaluation target is the same as the polymer having a hydrophobic portion described above.

In this reagent, the concentration of the fluorescent dye is preferably within a range from 1 to 1,000 µg/mL.

The osmotic pressure of this reagent is preferably within a range from 100 to 400 mosm/Kg. This ensures that penetration of the reagent itself into the cells is suppressed, and the polymer having a hydrophobic portion can be stained selectively, which can assist in evaluating the existence of polymers having a hydrophobic portion within the cell suspension, or the amount of those polymers.

The osmotic pressure of this reagent is preferably at least 100 mosm/Kg, more preferably at least 200 mosm/Kg, and even more preferably 250 mosm/Kg or greater.

The osmotic pressure of this reagent is preferably not more than 400 mosm/Kg, more preferably not more than 375 mosm/Kg, and even more preferably 350 mosm/Kg or less.

Here, the osmotic pressure describes the pressure which, when water as a solvent is placed on one side of a semipermeable membrane and the solution is placed on the other side, must be applied on the solution side to prevent migration of the water through the semipermeable membrane and into the solution side.

One embodiment is able to provide the use of a reagent, which contains a fluorescent dye that is capable of staining a polymer having a hydrophobic portion and a buffer solution, and has an osmotic pressure within a range from 100 to 400 mosm/Kg, for the purpose of evaluating a cell suspension.

### <Microcarrier Evaluation Method> (not covered by the subject-matter of the claims)

A method for evaluating microcarriers according to one embodiment is characterized by including: preparing a sample which either contains a liquid and microcarriers for use in cell culture, or contains a liquid obtained following treatment of the microcarriers, adding a fluorescent dye that is capable of staining a polymer having a hydrophobic portion to the sample and performing fluorescence analysis, and evaluating the microcarriers based on the fluorescence analysis of the sample.

This enables microcarriers to be provided that has been evaluated for the existence or absence of contamination with polymer fragments. For example, by using this evaluation method to first confirm that the amount of polymers having a hydrophobic portion and having a small particle size within the microcarriers are low, and then preparing a cell suspension using this microcarrier, the amount of polymers having a hydrophobic portion of small particle size within the obtained cell suspension can be reduced.

The microcarriers used in a cell culture may be an unused microcarrier, or may be microcarriers that has been recovered following a cell culture for reuse. Either type of microcarrier may be used as the microcarriers of the evaluation target. In one embodiment, microcarriers formed using a hydrophobic polymer or a polymer having hydrophobic groups can be evaluated favorably.

In the sample containing the microcarriers and the liquid, the liquid is preferably an aqueous liquid, and preferably contains water as the main solvent.

Samples containing a liquid obtained following treatment of microcarriers include, for example, liquids obtained by dispersing microcarriers raw material in a liquid, and then removing the microcarriers. A specific example is a liquid obtained following the washing of microcarriers with a liquid and subsequent recovery of the microcarriers. This liquid is preferably an aqueous liquid, and preferably contains water as the main solvent.

In the sample, there are no particular limitations on the microcarriers concentration, and the concentration may be within a range from 0.1 to 100 g/L, from 1 to 100 g/L, from 1 to 50 g/L, or from 1 to 20 g/L. In those cases where the microcarriers concentration in the sample is high, the sample may be diluted with additional liquid to bring the concentration within the range suitable for fluorescence analysis.

Any of the fluorescent dyes described above may be used as the fluorescent dye that is added to the sample. In the method for evaluating the microcarriers, because no cells exists in the sample, any fluorescent dye capable of staining polymers having a hydrophobic portion can be used favorably. From the viewpoints of versatility and fluorescent strength, a coumarin dye or DCM dye can be used particularly favorably.

The method used for adding the fluorescent dye and the fluorescence analysis method may employ the methods described above.

A method for evaluating microcarriers by adding a fluorescent dye to the sample, irradiating the sample with an excitation light, and then analyzing the fluorescence is described below.

There are no particular limitations on the method used for analyzing the fluorescence of the sample containing the added fluorescent dye.

In those cases where an excitation light is irradiated onto the sample containing the added fluorescent dye, the existence of a polymer having a hydrophobic portion within the sample can be confirmed by observing the fluorescence in the sample.

Furthermore, by analyzing the fluorescence from the sample, differences in the fluorescent intensity can be used to contrast and evaluate the amounts of polymers having a hydrophobic portion.

By analyzing the fluorescence from the sample, the existence of a polymer having a hydrophobic portion or the amount of that polymer can be evaluated, but the size, shape, or concentration or the like of the polymer having a hydrophobic portion, or a combination of these properties, may also be measured. For example, a fluorescence microscope or spectrofluorophotometric analysis may be used to conduct the measurement. Details are described above in relation to the measurement method.

The method for evaluating microcarriers may include, for example, evaluating the existence of a polymer having a hydrophobic portion with a particle size of 1 µm or smaller within the sample, or evaluating the amount of the polymer. The average particle size of a typical microcarrier is within a range from 120 to 250 µm, and therefore the existence of small fragments with a small particle size of 1 µm or smaller, or the amount of such fragments, may be evaluated.

In those cases where an evaluation is conducted using a sample containing microcarriers and a liquid, the sample contains the microcarriers, and may also contain polymers having a hydrophobic portion of small particle size. Accordingly, when a fluorescent dye is added to the sample, fluorescence from the microcarriers can be detected, but there is a possibility that fluorescence may also be emitted from the polymers having a hydrophobic portion of small particle size. In such cases, by using a fluorescence microscope or the like to conduct an image observation, the microcarriers and the polymers having a hydrophobic portion of small particle size can be distinguished, thus enabling evaluation of the existence of polymers having a hydrophobic portion of small particle size.

In those cases where evaluation is conducted using, as an example of a sample containing a liquid obtained following treatment of microcarriers, a sample prepared by removing the microcarriers from the liquid, the sample contains no microcarrier, but may contain polymer having a hydrophobic portions with particle sizes smaller than the microcarriers. Accordingly, by adding a fluorescent dye to the sample and detecting fluorescence, an evaluation can be conducted as to whether or not a polymer having a hydrophobic portion of small particle size exists within the sample. Based on the evaluation results, an evaluation can also be made that the microcarriers subjected to the treatment probably contained the polymer having a hydrophobic portion of small particle size. Further, by observing the sample containing the added fluorescent dye with a fluorescence microscope, the shape, distribution, and concentration and the like of the polymer having a hydrophobic portion can be evaluated, and the existence of the polymer having a hydrophobic portion of small particle size probably contained within the microcarriers of the treatment target can be evaluated. Furthermore, by measuring the fluorescent intensity from the sample containing the added fluorescent dye, the amount and concentration and the like of the polymer having a hydrophobic portion of small particle size probably contained within the microcarriers of the treatment target can be evaluated.

### <Cell Suspension Production Method> (not covered by the subject-matter of the claims)

One example of a method for producing a cell suspension, using the microcarrier evaluation method, the cell suspension evaluation method, or a combination thereof, is described below.

A production method (A) for a cell suspension according to one example includes washing and filtering microcarriers used for cell culture, adding a fluorescent dye capable of staining a polymer having a hydrophobic portion to the filtered filtrate and performing fluorescence analysis, evaluating the microcarriers based on the fluorescence analysis of the filtrate, and conducting cell culture using the evaluated microcarrier.

A production method (B) for a cell suspension according to another example includes conducting cell culture using a composition containing microcarriers, removing the microcarriers from the cell suspension containing the cells and the microcarriers following cell culture, adding a fluorescent dye capable of staining a polymer having a hydrophobic portion to the cell suspension from which the microcarriers have been removed and performing fluorescence analysis, and evaluating the cell suspension based on the fluorescence analysis of the cell suspension from which the microcarriers have been removed.

A production method (C) for a cell suspension according to yet another example includes using microcarriers evaluated by the production method (A) described above to conduct a cell culture and obtain a cell suspension, removing the microcarriers from the cell suspension containing the cells and the microcarriers following cell culture, adding a fluorescent dye capable of staining a polymer having a hydrophobic portion to the cell suspension from which the microcarriers have been removed and performing fluorescence analysis, and evaluating the cell suspension based on the fluorescence analysis of the cell suspension from which the microcarriers have been removed.

By using these embodiments, a cell suspension can be provided for which the existence or absence of contamination of the cell suspension with polymers having a hydrophobic portion has been evaluated.

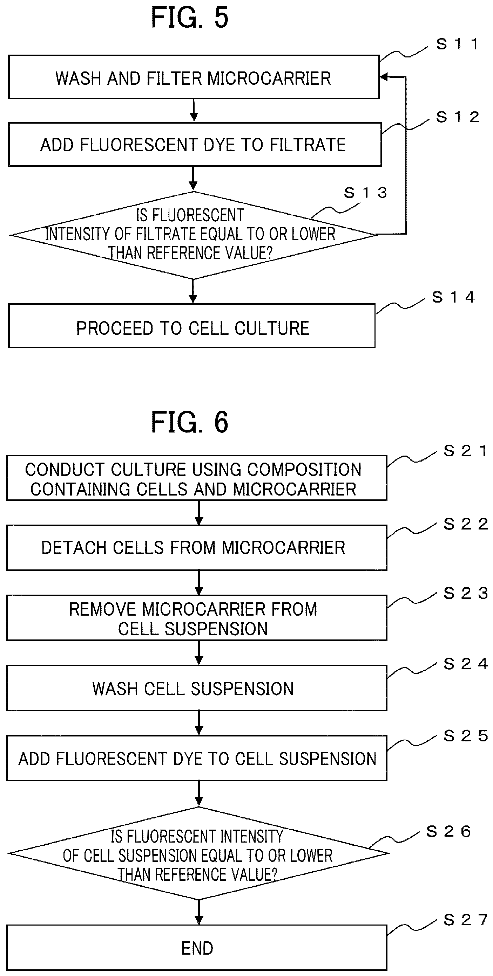

The production method (A) for a cell suspension is described below using a flowchart illustrated in FIG. 5.

First, in a step (S11) of washing and filtering the microcarriers, microcarriers in a powdered form or dispersed liquid form is mixed and stirred with a liquid to wash the microcarriers, and following washing, a filtration is performed using a filter or the like to separate the microcarriers and the filtrate. For example, in order to separate microcarriers of typical size, a filter with a pore size of 1 to 100 µm, and preferably 10 to 50 µm, may be used.

Subsequently, in a step (S12) of adding a fluorescent dye to the filtrate, the fluorescent dye is added to the filtrate. The fluorescent dye is preferably a dye that is capable of staining a polymer having a hydrophobic portion, and for example, the types of fluorescent dyes described above may be used. Further, there are no particular limitations on the method used for adding the fluorescent dye to the filtrate, and any of the methods described above may be used.

Next, in a step (S13) of determining whether the fluorescent intensity of the filtrate containing the added fluorescent dye is equal to or below a prescribed reference value, an excitation light is irradiated onto the filtrate containing the added fluorescent dye, the fluorescent intensity is measured, and a determination is made as to whether the measured value is equal to or lower than the reference value. A low value for this fluorescent intensity indicates that the amount of polymers having a hydrophobic portion contained within the filtrate is small, and as a result, an evaluation can be made that the amount of small fragments of polymers having a hydrophobic portion contained within the microcarriers are low. Following confirmation that the microcarriers either contain no small fragments of polymers having a hydrophobic portion, or contains only a small amount of these small fragments of polymers having a hydrophobic portion, by subsequently supplying this microcarrier to a cell culture, contamination of the finally obtained cell suspension with small fragments of polymers having a hydrophobic portion can be better prevented.

The reference value in S13 may be set so as to satisfy appropriate standards required in various types of cell culture. In one example, the fluorescent intensity of water containing a concentration of 10% by mass, and preferably 1% by mass, of polymers having a hydrophobic portion that have been obtained by crushing the microcarriers supplied to the washing process to a size of 1 µm or smaller may be used as the reference value. In another example, the fluorescent intensity of water containing 10 g/L, and preferably 1 g/L, of polystyrene with an average particle size of 1 µm may be used as the reference value. In yet another example, observation using a fluorescence microscope may be used, and the state in which the number of polymer fragments having a particle size of 1 µm or smaller per unit of surface area is 10 fragments/m² may be set as the reference value.

In S13, instead of using the method in which an excitation light is irradiated onto the filtrate containing the added fluorescent dye and the fluorescent intensity is then measured, a method may be used in which an excitation light is irradiated onto the filtrate containing the added fluorescent dye and the fluorescence is then detected by either fluorescence microscope or visual observation. Further, an excitation light may be irradiated onto the filtrate containing the added fluorescent dye, a fluorescence microscope then used to measure the shape, size, and/or concentration and the like of polymers having a hydrophobic portion, and these results then used to analyze the state of contamination of the filtrate with polymers having a hydrophobic portion. A decision as to whether or not to supply the microcarriers to cell culture can then be made on the basis of these observation results.

Subsequently, in those cases where the reference value is satisfied in S13, the microcarriers can be supplied to the cell culture (S14). In those cases where the reference value is not satisfied in S13, the process may return to S11, and washing may be repeated until the reference value is satisfied.

Next, the production method (B) for a cell suspension is described below using a flowchart illustrated in FIG. 6.

First, in a step (S21) of conducting culturing using a composition containing cells and microcarriers, a cell culture may be conducted in accordance with normal methods using the composition containing the cells and the microcarriers.

Subsequently, in a step (S22) of detaching the cells from the microcarriers, the cells may be detached from the microcarriers in accordance with typical methods. For example, a protease such as trypsin may be added to the cell suspension to detach the cells from the microcarriers.

Next, in a step (S23) of removing the microcarriers from the cell suspension, the cells and the microcarriers can be separated in accordance with typical methods. For example, by using a filter or the like to remove coarse particles having a particle size larger than the cells, the cells and the microcarriers can be separated. For example, in order to remove microcarriers of typical size, a filter with a pore size of 10 to 150 µm, and preferably 50 to 100 µm, may be used. In this method, small particles having a particle size the same as, or smaller than, the size of the cells are not removed, and therefore the cells and small fragments of polymers having a hydrophobic portion are not separated, meaning there is a possibility that fragments of polymers having a hydrophobic portions may also remain in the cell suspension together with the cells. Further, in those cases where the specific gravities of the cells and the microcarriers differ, separation may also be conducted by centrifugal separation.

Subsequently, a step (S24) of washing the cell suspension is an optional step and may be provided as necessary. In this step S24, for example, small particles that may be contained in the cell suspension can be removed by substituting the dispersion medium of the cell suspension. There is a possibility that the cell suspension may contain fragments of polymers having a hydrophobic portion or the like, which may be incorporated within the microcarrier raw material itself. These types of fragments of polymers having a hydrophobic portion with a particle size smaller than the cells can be removed by washing.

By executing S23 and D24, coarse particles larger than the cells and small particles smaller than the cells can be removed, but particles of the same size as the cells or of similar size to the cells are unable to be removed from the cell suspension.

Next, in a step (S25) of adding a fluorescent dye to the filtrate, a fluorescent dye is added to the cell suspension. The fluorescent dye is preferably a dye that is capable of staining a polymer having a hydrophobic portion, and for example, the types of fluorescent dyes described above may be used. Further, there are no particular limitations on the method used for adding the fluorescent dye to the filtrate, and any of the methods described above may be used.

Next, in a step (S26) of determining whether the fluorescent intensity of the cell suspension containing the added fluorescent dye is equal to or below a prescribed reference value, an excitation light is irradiated onto the cell suspension containing the added fluorescent dye, the fluorescent intensity is measured, and a determination is made as to whether the measured value is equal to or lower than the reference value. A low value for this fluorescent intensity indicates that the amount of polymers having a hydrophobic portion contained within the cell suspension is small.

The reference value in S26 may be set so as to satisfy appropriate standards required in various types of cell culture. In one example, the fluorescent intensity of water containing a concentration of 10% by mass, and preferably 1% by mass, of polymers having a hydrophobic portion that have been obtained by crushing the microcarriers supplied to the cell culture to a size of 1 µm or smaller may be used as the reference value. In another example, the fluorescent intensity of water containing 10 g/L, and preferably 1 g/L, of polystyrene with an average particle size of 1 µm may be used as the reference value. In yet another example, observation using a fluorescence microscope may be used, and the state in which the number of polymer fragments having a particle size of 1 µm or smaller per unit of surface area is 10 fragments/m² may be set as the reference value.

In S26, instead of using the method in which an excitation light is irradiated onto the cell suspension containing the added fluorescent dye and the fluorescent intensity is then measured, a method may be used in which an excitation light is irradiated onto the cell suspension containing the added fluorescent dye and the fluorescence is then detected by either fluorescence microscope or visual observation. Further, an excitation light may be irradiated onto the cell suspension containing the added fluorescent dye, a fluorescence microscope then used to measure the shape, size, and/or concentration and the like of polymers having a hydrophobic portion, and these results then used to analyze the state of contamination of the cell suspension with polymers having a hydrophobic portion. Based on these observation results, the existence or absence of contamination of the cell suspension with polymers having a hydrophobic portion can also be evaluated. Furthermore, by using these observation results, a decision may also be made to conduct further removal of polymers having a hydrophobic portion from the cell suspension.

One example of a methods for culturing cells using microcarriers is described below.

The cell culture method according to one embodiment may include preparing microcarriers, disposing the microcarriers in a culture system, and culturing the cells in the presence of the microcarriers.

In the step of disposing the microcarriers in the culture system, for example, the culture system may first be inoculated with the target cells, and the microcarriers may then be disposed in the culture system.

Examples of cells that may be used as the culture target cells include cells for which growth can be accelerated when supported on the microcarriers, and for example, adherent cells are preferred. The cells may be somatic cells, and for example, may be stem cells, precursor cells, or differentiated cells or the like, but stem cells are preferred.

The cells are preferably animal-derived cells, and are more preferably mammalian-derived cells. Examples of the mammals include humans, monkeys, chimpanzees, cows, pigs, horses, sheep, goats, rabbits, rats, mice, marmots, dogs and cats. There are no particular limitations on the tissue from which the cells are derived, and examples include the skin, liver, kidneys, muscles, bone, blood vessels, blood, or nerve tissue. The cells may be primary culture cells, cultured cell lines, or recombinant culture cell lines or the like.

Examples of the stem cells include somatic stem cells such as mesenchymal stem cells, hematopoietic stem cells, neural stem cells, myeloid stem cells, germline stem cells and dental pulp stem cells, and mesenchymal stem cells are preferred. The term "mesenchymal stem cells" refers broadly to somatic stem cells which exist in various tissues in the body, and can be differentiated into all or some mesenchymal cells such as osteoblasts, chondrocytes and adipocytes. Examples of mesenchymal stem cells include bone marrow-derived mesenchymal stem cells, umbilical cord-derived mesenchymal stem cells and adipose tissue-derived mesenchymal stem cells, and bone marrow-derived mesenchymal stem cells are preferred.

Pluripotent stem cells such as induced pluripotent stem cells (iPS cells), embryonic stem cells (ES cells), embryonic germ cells (EG cells), multipotent germ stem cells (mGS cells), embryonic carcinoma cells (EC cells) and Muse cells may be used as the stem cells.

The cells may be differentiated cells such as endothelial cells, epidermal cells, epithelial cells, myocardial cells, myoblasts, nerve cells, bone cells, osteoblasts, fibroblasts, adipose cells, hepatic cells, renal cells, pancreatic cells, adrenal gland cells, periodontal ligament cells, gingival cells, periosteal cells, skin cells, dendritic cells, macrophages and lymphocyte cells; or precursor cells that represent a preliminary stage between stem cells and these differentiated cells.

A single type of the above cells may be used alone, or combination of two or more types of cell may be used.

The culture medium used for culturing the cells preferably contains an inorganic salt, an amino acid, sugar and water. The medium may also contain optional components such as serum, vitamins, hormones, antibiotics, growth factors and adhesion factors. Media that are known as basal media for cell culture may be used as the medium.

In other words, any medium that is known for use in culturing the selected cells may be used as the medium without any particular limitations, and examples include, but are not limited to, DMEM (Dulbecco's Modified Eagle's Medium), MEM (Eagle's Minimum Essential Medium), and αMEM medium (α-Modified Eagle's Minimum Essential Medium).

In those cases where a serum is added to the culture medium, serums such as Fetal bovine serum (FBS), horse serum and human serum may be used.

The medium used for the culture may be free of xenogeneic components. Media that are free of xenogeneic components may include serum substitute additives (for example, Knockout Serum Replacement (KSR) (manufactured by Invitrogen Corporation), Chemically-defined Lipid Concentrate (manufactured by Gibco Inc.), and Glutamax (manufactured by Gibco Inc.) and the like) instead of animal-derived serum.

In addition, serum-free media such as Essential 8 (manufactured by Thermo Fisher Scientific Inc.), mTeSR1 (manufactured by STEMCELL Technologies Inc.), the StemFit series (manufactured by Takara Bio Inc.) and StemFlex (manufactured by Thermo Fisher Scientific Inc.) may also be used.

Additives may be added to the medium if necessary. Examples of these additives include vitamins such as vitamin A, vitamin B1, vitamin B2, vitamin B6, vitamin B12, vitamin C and vitamin D; coenzymes such as folic acid; amino acids such as glycine, alanine, arginine, asparagine, glutamine, isoleucine and leucine; sugars and organic acids that act as carbon sources such as lactic acid; growth factors such as EGF, FGF, PFGF and TGF-β; interleukins such as IL-1 and IL-6; cytokines such as TNF-α, TNF-β and leptin; metal transporters such as transferrin; metal ions such as iron ions, selenium ions and zinc ions; SH reagents such as β-mercaptoethanol and glutathione; and proteins such as albumin.

There are no particular limitations on the cell culture method, and a method suited to the particular cell may be used. Typically, the cell culture may be conducted in an environment having a temperature within a range from 30 to 40°C, and preferably a temperature of 36 to 37°C, a pH within a range from 6.2 to 7.7, and preferably at a pH of 7.4, and a CO₂ concentration within a range from 4 to 10% by volume, and preferably 5 to 7% by volume.

In the case of culturing using microcarriers, although dependent on the cell type and the type of microcarrier, for a cell suspension containing, for example, 1×10³ to 20×10⁵ cells/mL, and preferably 5×10³ to 10×10⁵ cells/mL, an amount of the microcarriers within a range from 0.1 to 100 g/L, and preferably 1.0 to 20 g/L, may be combined with the cells.

A second embodiment may include the various embodiments described below.

An embodiment (1) is a method that includes evaluation of a cell suspension, the method including adding a fluorescent dye capable of staining a polymer having a hydrophobic portion to a cell suspension containing cells, irradiating the cell suspension containing the added fluorescent dye with an excitation light and performing fluorescence analysis, and then evaluating the cell suspension based on the fluorescence analysis.

An embodiment (2) is a method that includes evaluating microcarriers, the method including preparing a sample which either contains a liquid and microcarriers for use in cell culture, or contains a liquid obtained following treatment of the microcarriers, adding a fluorescent dye that is capable of staining a polymer having a hydrophobic portion to the sample and performing fluorescence analysis, and evaluating the microcarriers based on the fluorescence analysis.

An embodiment (3) is a cell suspension containing cells, wherein the number of polymers having a hydrophobic portion and a size of 1 µm or smaller is 10 or fewer per 1×10⁴ cells.

An embodiment (4) is a cell suspension containing cells and a fluorescent dye that is capable of staining a polymer having a hydrophobic portion, wherein the fluorescent intensity emitted due to staining of the polymer having a hydrophobic portion by the fluorescent dye is higher than the fluorescent intensity emitted due to staining of the cells by the fluorescent dye.

An embodiment (5) is a reagent for evaluating a cell suspension, the reagent containing a fluorescent dye that is capable of staining a polymer having a hydrophobic portion and a buffer solution, wherein the osmotic pressure of the reagent is within a range from 100 to 400 mosm/Kg.

An embodiment (6) relates to the use of a reagent for evaluating a cell suspension, wherein the reagent contains a fluorescent dye that is capable of staining a polymer having a hydrophobic portion and a buffer solution, and has an osmotic pressure within a range from 100 to 400 mosm/Kg.

In the embodiment (1), (2), (5) or (6), the fluorescent dye may contain at least one dye selected from the group consisting of porphyrin dyes, phthalocyanine dyes, poly(phenylene vinylene) dyes, pyrene dyes, xanthene dyes, coumarin dyes and DCM dyes.

In the embodiment (1), (2), (5) or (6), the fluorescent dye may have different fluorescent characteristics in intracellular environments and extracellular environments.

In the embodiment (1), (2), (5) or (6), the fluorescent intensity emitted in the cell suspension due to staining of the polymer having a hydrophobic portion by the fluorescent dye may be higher than the fluorescent intensity emitted in the cell suspension due to staining of the cells by the fluorescent dye.

In the embodiment (1), the fluorescence analysis of the cell suspension may be used to evaluate the existence in the cell suspension of a polymer having a hydrophobic portion or the amount of that polymer.

In the embodiment (1), the fluorescence analysis of the cell suspension may include the measurement of at least one property selected from the group consisting of the size, shape and concentration of the polymer having a hydrophobic portion within the cell suspension.

In the embodiment (1), the fluorescence analysis of the cell suspension may be used to measure the particle size of the polymer having a hydrophobic portion, and evaluate the existence of a polymer having a hydrophobic portion with a particle size that is equal to or smaller than the size of the cells.

In the embodiment (1), the fluorescence analysis of the cell suspension may be used to measure the particle size of the polymer having a hydrophobic portion, and evaluate the existence of a polymer having a hydrophobic portion with a particle size that is 0.01 times to 5 times the size of the cells.

In the embodiment (1), in those cases where, in the cell suspension, at least one property selected from the group consisting of the temperature, concentration and pH of the cell suspension satisfies a certain range, the fluorescent intensity emitted within the cell suspension as a result of staining of a polymer having a hydrophobic portion with the fluorescent dye may be higher than the fluorescent intensity emitted within the cell suspension as a result of staining of the cells with the fluorescent dye.

In the embodiment (1), the cell suspension may be a cell suspension obtained by conducting cell culture using cells and microcarriers, separating the cells and the microcarriers following the cell culture, and then removing the microcarriers.

In the embodiment (1), the fluorescence analysis of the cell suspension may be used to measure the particle size of the polymer having a hydrophobic portion, and evaluate the existence of a polymer having a hydrophobic portion with a particle size that is equal to or smaller than the average particle size of the microcarriers.

In the embodiment (2), the fluorescence analysis of the sample may be used to evaluate the existence in the microcarriers of a polymer having a hydrophobic portion with a particle size of 1 µm or smaller, or the amount of that polymer.

In the embodiment (2), the sample may be a filtrate obtained by washing the microcarriers using a liquid and then performing a filtration.

In this description, numerical ranges indicated using the expression "a to b" indicate a range that includes the numerical values before and after the "to" as the minimum value and maximum value respectively. In the case of numerical ranges listed in a stepwise manner in this description, the upper limit value or lower limit value from the numerical range of any particular step may be arbitrarily combined with the upper limit value or lower limit value from the numerical range of another step. Unless specifically stated otherwise, for all materials exemplified in this description, a single material may be used alone, or a combination of two or more materials may be used. In this description, unless specifically stated otherwise, the amount of each component in a composition, in the case where a plurality of substances corresponding with any particular component exist in the composition, means the total amount of the plurality of substances that exist in the composition. The term "step" refers to not only independent steps, but also includes steps that achieve an intended action, even if the step cannot be clearly differentiated from another step.

### EXAMPLES

The disclosure is described below in further detail using a series of examples, but provided the technical scope of the disclosure is not exceeded, the invention is not limited to these examples.

### <Production Example 1>

A series of aqueous and PBS solutions of pectin (hereafter referred to as "pectin solutions") were prepared with the concentrations shown in Table 1, and using each solution, a series of mixtures were prepared by placing 0.5 mL of the pectin solution, 0.5 mL of concentration hydrochloric acid (12 mol/L) and 10 mg of naphthoresorcinol in a screwcap test tube, and each mixture was heated under stirring at 90°C for 2 hours to obtain a reaction mixture.

Following cooling of the reaction mixture to room temperature, 1.0 mL of butyl acetate was added to the cooled reaction mixture and stirred for one minute. Subsequently, the water phase was removed from the sample, obtaining a mixture of the organic phase and insoluble matter.

Subsequently, 1.0 mL of ethanol was added to the obtained mixture and stirred to obtain a uniform solution.

Following dilution of the obtained solution a further 100-fold with ethanol, the fluorescent intensity at an excitation wavelength of 512 nm was measured. A fluorescence spectrophotometer (manufactured by JASCO Corporation) was used for the fluorescence analysis. The results are shown in Table 1.

**[Table 1]**

| Pectin concentration [µg/mL] | Fluorescent intensity [a.u.] |
|---|---|
| 10 | 14.44 |
| 20 | 30.95 |
| 40 | 64.75 |
| 80 | 113.65 |

Based on the results in Table 1, it was evident that fluorescence analysis of the pectin in the samples was possible, and that by using a large amount of naphthoresorcinol relative to the amount of pectin, the amount of pectin could be quantified. Further, it was evident that the pectin concentration exhibited a proportional relationship with the fluorescent intensity, meaning that by using a calibration curve or the like, the pectin concentration could be confirmed from the fluorescent intensity. By using this method, and conducting either fluorescence analysis or absorption analysis of a saccharide, the saccharide in a sample can be measured or quantified simply and with good sensitivity. Consequently, even in the case of a polysaccharide, by employing a combination of hydrolysis and either fluorescence analysis or absorption analysis, the hydrolysate of the polysaccharide can be measured simply and with good sensitivity.

Accordingly, by applying the measurement method of the disclosure, a cell suspension obtained by conducting cell culture using a dissolvable polysaccharide cell support and subsequently dissolving the cell support and collecting the cells can be evaluated easily by using the existence or absence of cell support residues or the amount of those residues as indicators.

### <Production Example 2: Cell Suspension Evaluation Method>

### [Preparation of Dye Solution, Cell Suspension, Microcarrier Fragment Dispersion]

The dye powders and dye stock solutions listed below were each diluted with phosphate buffered saline solution (PBS) to prepare dye solutions having concentrations within a range from 1 µg/mL to 1,000 µg/mL

A cell suspension of human mesenchymal stem cells (hMSC, sourced from Takara Bio Inc.) was prepared by collecting cultured hMSC by trypsin treatment and then performing a centrifugal separation and PBS washing. The cell density was 8.2×10⁵ cells/mL.

Microcarriers fragment dispersion was prepared by placing 5 g of microcarriers ("SoloHill Star-Plus", diameter: 125 to 212 µm, a spherical crosslinked polystyrene, manufactured by Pall Corporation) in a glass vial, crushing the microcarriers with a magnetic stirring bar for one day, filtering the mixture through a 60 µm mesh and collecting the filtrate. In other words, a dispersion was prepared that contained microcarrier fragments of small particle size that had passed through the 60 µm mesh.

### [Dye Solutions]

The dyes used, and the concentrations of the dye solutions were as follows.
Dye (a1): tetraphenylporphyrin tetrasulfonic acid (TPPS); 1 µg/mL
Dye (a2): poly(5-methoxy-2-(3-sulfopropoxy)-1,4-phenylenevinylene; 100 µg/mL
Dye (a3): methoxypolyethylene glycol pyrene; 1,000 µg/mL
Dye (a4): naphthofluorescein; 10 µg/mL
Dye (a5): BD140; 1 µg/mL
Dye (a6): coumarin 6; 1 µg/mL
Dye (a7): DCM (4-(dicyanomethylene)-2-methyl-6-(4-dimethylaminostyryl)-4H-pyran); 1 µg/mL

### [Staining and Observation]

First, 500 µL of the cell suspension and 50 µL of the microcarrier fragment dispersion were mixed in a 24-well plate, 50 µL of the dye solution was then added, and following stirring, the mixture was left to stand for 10 minutes. Subsequently, the obtained mixture was observed as a bright field image and a fluorescence image using a fluorescence microscope.

In order to prepare blank samples, 500 µL of the cell suspension and 50 µL of PBS (phosphate buffered physiological saline solution) were mixed in a 24-well plate, 50 µL of the dye solution was then added, and following stirring, the mixture was left to stand for 10 minutes. Subsequently, the obtained mixture was observed as a bright field image and a fluorescence image using a fluorescence microscope.

Blue fluorescence was observed with an excitation light of 360 to 370 nm, green fluorescence was observed with an excitation light of 450 to 480 nm, and red fluorescence was observed with an excitation light of 510 to 550 nm.

The dye (a1) was observed as red fluorescence, the dye (a2) was observed as green fluorescence, the dye (a3) was observed as blue fluorescence and red fluorescence, the dye (a4) was observed as green fluorescence and red fluorescence, the dye (a5) was observed as red fluorescence, the dye (a6) was observed as green fluorescence, and the dye (a7) was observed as red fluorescence.

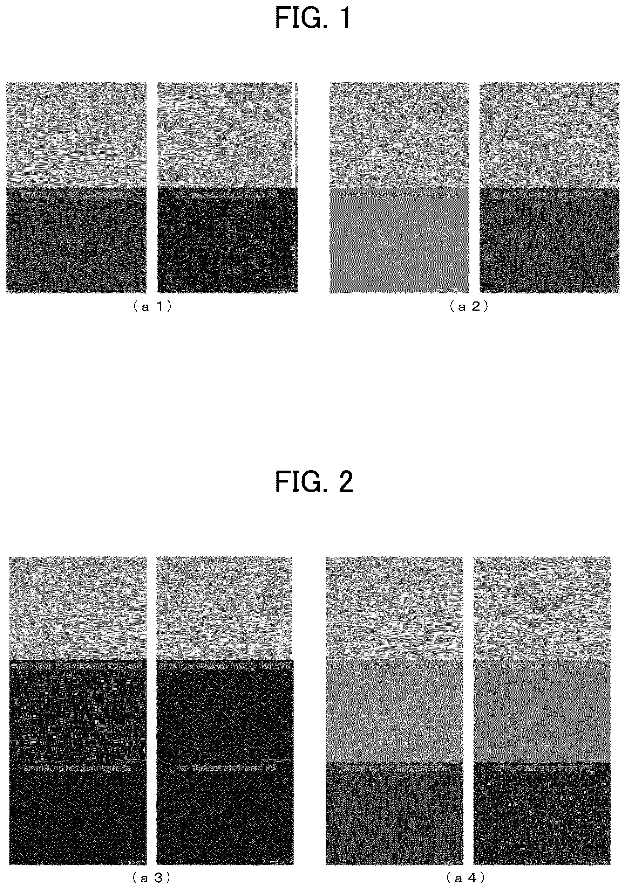

The obtained bright field images and fluorescence images are shown in FIG. 1. In FIG. 1, the left column of images represent the blank samples containing the cells and the fluorescent dye, whereas the right column of images represent the samples containing the cells, the fluorescent dye, and the microcarrier fragments. In FIG. 1, the upper row of images are bright field images and the lower row of images are fluorescence images.

In FIG. 1, (a1) is an example using the dye (a1), and the lower row of images are red fluorescence observation images. There was almost no fluorescence from the cells, but strong red fluorescence was observed from the microcarrier fragments.

In FIG. 1, (a2) is an example using the dye (a2), and the lower row of images are green fluorescence observation images. There was almost no fluorescence from the cells, but strong green fluorescence was observed from the microcarrier fragments.

More of the obtained bright field images and fluorescence images are shown in FIG. 2. In FIG. 2, the left column of images represent the blank samples containing the cells and the fluorescent dye, whereas the right column of images represent the samples containing the cells, the fluorescent dye, and the microcarrier fragments. In FIG. 2, the top row of images are bright field images, the middle row of images are blue (a3) or green (a4) fluorescence images, and the bottom row of images are red fluorescence images.

In FIG. 2, (a3) is an example using the dye (a3). When the samples were excited with light with a wavelength of 360 to 370 nm, the cells and the microcarrier fragments emitted blue fluorescence, whereas when the samples were excited with light with a wavelength of 510 to 550 nm, there was no fluorescence from the cells, but red fluorescence was observed from the microcarrier fragments.

In FIG. 2, (a4) is an example using the dye (a4). When the samples were excited with light with a wavelength of 450 to 480 nm, the cells and the microcarrier fragments emitted green fluorescence, whereas when the samples were excited with light with a wavelength of 510 to 550 nm, there was no fluorescence from the cells, but red fluorescence was observed from the microcarrier fragments.

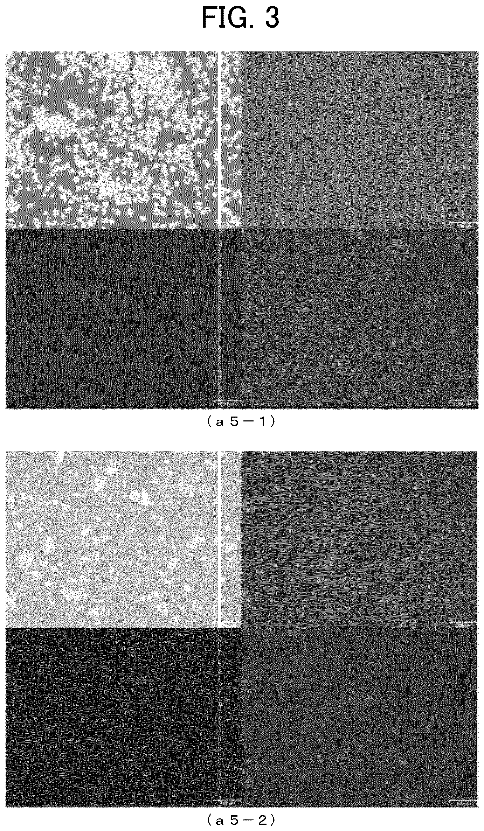

Bright field images and fluorescence images obtained by observations using the dye (a5) are shown in FIG. 3. In FIG. 3, the upper set of images (a5-1) represent blank samples containing the cells and the fluorescent dye, whereas the lower set of images (a5-2) represent the samples containing the cells, the fluorescent dye, and the microcarrier fragments. In FIG. 3, for both the sample (a5-1) and the sample (a5-2), the upper left image is a bright field image, the lower left image is a blue fluorescence image, the upper right image is a pink fluorescence image, and the lower right image is a red fluorescence image.

With the dye (a5), both the cells and the microcarrier fragments were observed in the pink fluorescence image and the red fluorescence image. In the pink and red fluorescence images, the cells and the microcarrier fragments can be differentiated and distinguished based on shape. With the dye (a5), in the blue fluorescence image, the microcarrier fragments were observed, albeit with a low fluorescent intensity. Based on the blue fluorescence image, a determination can be made of the existence or absence of microcarrier fragments.

Similarly, in the cases of the dye (a6) and the dye (a7), the cells and the microcarrier fragments can be differentiated and distinguished based on shape in the fluorescence images.

### <Production Example 3: Evaluation of Microcarrier Prior to Cell Culture>

### [Washing of Microcarrier]

Microcarriers ("SoloHill Star-Plus", diameter: 125 to 212 µm, a spherical crosslinked polystyrene, manufactured by Pall Corporation) was prepared.

Subsequently, a washing operation was conducted by mixing 3 g of this microcarrier and 25 mL, and then filtering the mixed liquid using a filter with a pore size of 10 µm. This washing operation was repeated three times.

### [Dye Solutions]

The fluorescent dyes used, and the concentrations of the dye solutions were as follows.
Dye (b1): tetraphenylporphyrin tetrasulfonic acid (TPPS); 1 µg/mL
Dye (b2): coumarin 6; 1 µg/mL
Dye (b3): DCM (4-(dicyanomethylene)-2-methyl-6-(4-dimethylaminostyryl)-4H-pyran); 1 µg/mL

### [Staining and Observation]

First, 50 µL of the dye solution was added to 500 µL of the filtrate obtained following completion of the three washing operations of the microcarriers, and following stirring, the mixture was left to stand for 10 minutes. Subsequently, the obtained mixture was observed as a bright field image and a fluorescence image using a fluorescence microscope.

Green fluorescence was observed with an excitation light of 450 to 480 nm, and red fluorescence was observed with an excitation light of 510 to 550 nm.

The dye (b1) was observed as red fluorescence, the dye (b2) was observed as green fluorescence, and the dye (b3) was observed as red fluorescence.

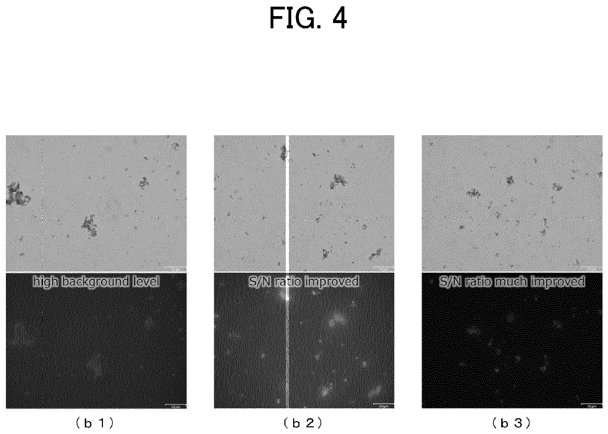

The obtained bright field images and fluorescence images are shown in FIG. 4. In FIG. 4, the upper row of images are bright field images and the lower row of images are fluorescence images.

In FIG. 4, (b1) is an example using the dye (b1), and the lower row of images are red fluorescence observation images. With the dye (b1), the microcarrier fragments observed in the bright field image of the upper row were also observed in the fluorescence image of the lower row, and the microcarrier fragments were able to be confirmed by the red fluorescence.

In FIG. 4, (b2) is an example using the dye (b2), and the lower row of images are green fluorescence observation images. With the dye (b2), the microcarrier fragments observed in the bright field image of the upper row were also observed in the fluorescence image of the lower row, and the microcarrier fragments were able to be confirmed by the green fluorescence.

In FIG. 4, (b3) is an example using the dye (b3), and the lower row of images are red fluorescence observation images. With the dye (b3), the microcarrier fragments observed in the bright field image of the upper row were also observed in the fluorescence image of the lower row, and the microcarrier fragments were able to be confirmed by the red fluorescence.

Based on the fact that microcarrier fragments were able to be confirmed in the filtrate following washing of the microcarriers, an evaluation can be made that the microcarrier contains these fragments prior to washing. Further, based on the image observations, the size and distribution of these fragments in the microcarriers prior to washing can be evaluated.

## Claims

1. A method for measuring cell support-derived components in a liquid sample such as a cell suspension containing cells, an evaluation sample obtained from a cell suspension, a sample containing a liquid and microcarriers for use in cell culture, and a sample containing a liquid obtained following treatment of microcarriers, wherein the cell support-derived components are derived from a polysaccharide cell support used in cell culture, the method comprising:
preparing a mixture containing
the liquid sample,
an acid, and
at least one aromatic compound that can produce a fluorescent or light-absorbing compound as a result of a reaction with a saccharide under heating, wherein the aromatic compound has at least one functional group selected from the group consisting of a hydroxyl group, an amino group, a nitro group and a carbonyl group,
heating the mixture, and
subjecting a reaction product in the mixture following heating to fluorescence analysis or absorption analysis.

2. The method according to Claim 1, wherein the aromatic compound that can produce a fluorescent or light-absorbing compound as a result of a reaction with a saccharide under heating is resorcinol, orcinol, fluoroglucinol, naphthoresorcinol, 5-oxytetralone, or anthrone.

3. The method according to Claim 1 or 2, wherein the liquid sample is a cell suspension containing cells that are cultured in the presence of a dissolvable cell support and collected following dissolution of the cell support.

4. The method according to any of Claims 1 to 3, wherein the method comprises,
quantifying the reaction product in the mixture following heating using fluorescence analysis or absorption analysis, and
within the mixture, a mass of the aromatic compound that can produce a fluorescent or light-absorbing compound as a result of a reaction with a saccharide under heating is at least 200 times a mass of the polysaccharide cell support-derived components.

5. The method according to any one of Claims 1 to 4, wherein a concentration of the aromatic compound that can produce a fluorescent or light-absorbing compound as a result of a reaction with a saccharide under heating in the mixture is from 1 mg/mL to 100 mg/mL.

6. The method according to Claim 3, wherein the liquid sample is an evaluation sample obtained from the cell suspension.

7. The method according to Claim 3, wherein the liquid sample is an evaluation sample obtained from the cell suspension, and
a mass of the aromatic compound that can produce a fluorescent or light-absorbing compound as a result of a reaction with a saccharide under heating is at least 200 times a mass of saccharides produced by hydrolysis of the cell support.

8. The method according to Claim 7, wherein fluorescence analysis or absorption analysis is used for quantifying the reaction product.

9. The method according to any one of Claims 6 to 8, wherein a concentration of the aromatic compound that can produce a fluorescent or light-absorbing compound as a result of a reaction with a saccharide under heating in the mixture is from 1 mg/mL to 100 mg/mL.

## Patentansprüche

1. Verfahren zum Messen von aus Zellträger stammenden Komponenten in einer Flüssigprobe, wie beispielsweise einer Zellsuspension, die Zellen enthält, einer aus einer Zellsuspension erhaltenen Untersuchungsprobe, einer Probe, die eine Flüssigkeit und Mikroträger zur Verwendung in Zellkultur enthält, und einer Probe, die eine nach der Behandlung von Mikroträgern erhaltene Flüssigkeit enthält, wobei die aus Zellträger stammenden Komponenten von einem in Zellkultur verwendeten Polysaccharid-Zellträger stammen, wobei das Verfahren umfasst:
Herstellen eines Gemischs enthaltend
die Flüssigprobe,
eine Säure und
mindestens eine aromatische Verbindung, die infolge einer Reaktion mit einem Saccharid unter Erhitzen eine fluoreszierende oder lichtabsorbierende Verbindung erzeugen kann, wobei die aromatische Verbindung mindestens eine funktionelle Gruppe aufweist, die aus der Gruppe ausgewählt ist, die aus einer Hydroxylgruppe, einer Aminogruppe, einer Nitrogruppe und einer Carbonylgruppe besteht,
Erhitzen des Gemischs, und
Unterziehen eines Reaktionsprodukts im Gemisch nach dem Erhitzen einer Fluoreszenzanalyse oder einer Absorptionsanalyse.

2. Verfahren gemäß Anspruch 1, wobei die aromatische Verbindung, die infolge einer Reaktion mit einem Saccharid unter Erhitzen eine fluoreszierende oder lichtabsorbierende Verbindung erzeugen kann, Resorcin, Orcin, Fluoroglucinol, Naphthoresorcin, 5-Oxytetralon oder Anthron ist.

3. Verfahren gemäß Anspruch 1 oder 2, wobei die Flüssigprobe eine Zellsuspension ist, die Zellen enthält, die in Gegenwart eines löslichen Zellträgers kultiviert und nach Auflösung des Zellträgers gesammelt werden.

4. Verfahren gemäß einem der Ansprüche 1 bis 3, wobei das Verfahren umfasst:
Quantifizieren des Reaktionsprodukts in dem Gemisch nach dem Erhitzen mittels Fluoreszenzanalyse oder Absorptionsanalyse, und
innerhalb des Gemischs eine Masse der aromatischen Verbindung, die infolge einer Reaktion mit einem Saccharid unter Erhitzen eine fluoreszierende oder lichtabsorbierende Verbindung erzeugen kann, mindestens das 200-fache einer Masse der aus dem Polysaccharid-Zellträger stammenden Komponenten betragend.

5. Verfahren gemäß einem der Ansprüche 1 bis 4, wobei eine Konzentration der aromatischen Verbindung, die infolge einer Reaktion mit einem Saccharid unter Erhitzen in dem Gemisch eine fluoreszierende oder lichtabsorbierende Verbindung erzeugen kann, zwischen 1 mg/ml und 100 mg/ml liegt.

6. Verfahren gemäß Anspruch 3, wobei die Flüssigprobe eine aus der Zellsuspension erhaltene Untersuchungsprobe ist.

7. Verfahren gemäß Anspruch 3, wobei die Flüssigprobe eine aus der Zellsuspension erhaltene Untersuchungsprobe ist und
eine Masse der aromatischen Verbindung, die infolge einer Reaktion mit einem Saccharid unter Erhitzen eine fluoreszierende oder lichtabsorbierende Verbindung erzeugen kann, mindestens das 200-fache einer Masse von Sacchariden beträgt, die durch Hydrolyse des Zellträgers erzeugt werden.

8. Verfahren gemäß Anspruch 7, wobei zur Quantifizierung des Reaktionsprodukts eine Fluoreszenzanalyse oder eine Absorptionsanalyse verwendet wird.

9. Verfahren gemäß einem der Ansprüche 6 bis 8, wobei eine Konzentration der aromatischen Verbindung, die infolge einer Reaktion mit einem Saccharid unter Erhitzen in dem Gemisch eine fluoreszierende oder lichtabsorbierende Verbindung erzeugen kann, zwischen 1 mg/ml und 100 mg/ml liegt.

## Revendications

1. Procédé de mesure de composants dérivés d'un support cellulaire dans un échantillon liquide tel qu'une suspension cellulaire contenant des cellules, un échantillon d'évaluation obtenu à partir d'une suspension cellulaire, un échantillon contenant un liquide et des microsupports à utiliser en culture cellulaire, et un échantillon contenant un liquide obtenu après traitement des microsupports, dans lequel les composants dérivés d'un support cellulaire sont dérivés d'un support cellulaire polysaccharidique utilisé en culture cellulaire, le procédé comprenant :
la préparation d'un mélange contenant
l'échantillon liquide,
un acide, et
au moins un composé aromatique qui peut produire un composé fluorescent ou absorbant la lumière à la suite d'une réaction avec un saccharide sous chauffage, dans lequel le composé aromatique présente au moins un groupe fonctionnel choisi dans le groupe constitué par un groupe hydroxyle, un groupe amino, un groupe nitro et un groupe carbonyle,
le chauffage du mélange, et
la soumission d'un produit de réaction dans le mélange après chauffage à une analyse de fluorescence ou à une analyse d'absorption.

2. Procédé selon la revendication 1, dans lequel le composé aromatique qui peut produire un composé fluorescent ou absorbant la lumière à la suite d'une réaction avec un saccharide sous chauffage est le résorcinol, l'orcinol, le fluoroglucinol, le naphthorésorcinol, la 5-oxytétralone ou l'anthrone.

3. Procédé selon la revendication 1 ou 2, dans lequel l'échantillon liquide est une suspension cellulaire contenant des cellules qui sont cultivées en présence d'un support cellulaire soluble et collectées après dissolution du support cellulaire.

4. Procédé selon l'une quelconque des revendications 1 à 3, dans lequel le procédé comprend,
la quantification du produit de réaction dans le mélange après chauffage en utilisant une analyse de fluorescence ou une analyse d'absorption, et
dans le mélange, une masse du composé aromatique qui peut produire un composé fluorescent ou absorbant la lumière à la suite d'une réaction avec un saccharide sous chauffage est au moins 200 fois une masse des composants dérivés d'un support cellulaire polysaccharidique.

5. Procédé selon l'une quelconque des revendications 1 à 4, dans lequel une concentration du composé aromatique qui peut produire un composé fluorescent ou absorbant la lumière à la suite d'une réaction avec un saccharide sous chauffage dans le mélange est de 1 mg/mL à 100 mg/mL.

6. Procédé selon la revendication 3, dans lequel l'échantillon liquide est un échantillon d'évaluation obtenu à partir de la suspension cellulaire.

7. Procédé selon la revendication 3, dans lequel l'échantillon liquide est un échantillon d'évaluation obtenu à partir de la suspension cellulaire, et
une masse du composé aromatique qui peut produire un composé fluorescent ou absorbant la lumière à la suite d'une réaction avec un saccharide sous chauffage est au moins 200 fois une masse de saccharides produits par hydrolyse du support cellulaire.

8. Procédé selon la revendication 7, dans lequel une analyse de fluorescence ou une analyse d'absorption est utilisée pour quantifier le produit de réaction.

9. Procédé selon l'une quelconque des revendications 6 à 8, dans lequel une concentration du composé aromatique qui peut produire un composé fluorescent ou absorbant la lumière à la suite d'une réaction avec un saccharide sous chauffage dans le mélange est de 1 mg/mL à 100 mg/mL.
